# EUROPEAN PATENT SPECIFICATION

(11) **EP 4 271 372 B1**
(45) Date of publication and mention of the grant of the patent: **20.05.2026**
(21) Application number: 21847779.2
(22) Date of filing: 30.12.2021
(51) Int. Cl.: A61K 31/343, A61P 31/04, A61P 31/14, A61K 47/69, A23L 33/00, A23L 29/00

(54) **INCLUSION COMPLEXES OF USNIC ACID FOR USE IN THE TREATMENT OF INFECTIONS WITH A CORONAVIRUS**
EINSCHLUSSKOMPLEXE VON USNINSÄURE ZUR VERWENDUNG IN DER BEHANDLUNG VON INFEKTIONEN MIT EINEM CORONAVIRUS
COMPLEXES D'INCLUSION D'ACIDE USNIQUE POUR UNE UTILISATION DANS LE TRAITEMENT D'INFECTIONS PAR UN CORONAVIRUS

(30) Priority: 30.12.2020 IT 202000032909
(43) Date of publication of application: 08.11.2023
(73) Proprietor: Vestatis GmbH, 20097 Hamburg (DE)
(72) Inventor: CERANA, Giorgio Stefano, 20097 Hamburg (DE); BOS, Peter, 20097 Hamburg (DE); FRANCK, Peter, 20097 Hamburg (DE); BONER, Thomas Detlef, 20097 Hamburg (DE)
(74) Representative: Benedetto, Marco
(86) International application number: PCT/IB2021/062486
(87) International publication number: WO 2022/144835

(56) References cited:
- EP-A1- 0 256 566
- WO-A1-2021/100026
- WO-A1-2021/186347
- WO-A1-2022/034549
- CN-A- 108 635 592
- CN-B- 104 398 477
- US-B1- 10 463 590
- HANNU ELO ET AL: "Potent activity of the lichen antibiotic (+)-usnic acid against clinical isolates of vancomycin-resistant enterococci and methicillin-resistant Staphylococcus aureus", NATURWISSENSCHAFTEN, SPRINGER, BERLIN, DE, vol. 94, no. 6, 24 January 2007 (2007-01-24), pages 465 - 468, XP019510310, ISSN: 1432-1904, DOI: 10.1007/S00114-006-0208-9
- INGOLFSDOTTIR K: "Usnic acid", PHYTOCHEMISTRY, ELSEVIER, AMSTERDAM , NL, vol. 61, no. 7, 1 December 2002 (2002-12-01), pages 729 - 736, XP004394262, ISSN: 0031-9422, DOI: 10.1016/S0031-9422(02)00383-7
- MARIANE C B LIRA ET AL: "Inclusion complex of usnic acid with [beta]-cyclodextrin: characterization and nanoencapsulation into liposomes", JOURNAL OF INCLUSION PHENOMENA AND MACROCYCLIC CHEMISTRY, KLUWER ACADEMIC PUBLISHERS, DO, vol. 64, no. 3-4, 5 March 2009 (2009-03-05), pages 215 - 224, XP019679280, ISSN: 1573-1111
- GUTHAPPA ROOPA: "Docking studies of usnic acid and sodium usnate on SARS CoV-2 main protease and spike protein RBD", 13 July 2020 (2020-07-13), XP055840672, Retrieved from the Internet <URL:https://chemrxiv.org/engage/api-gateway/chemrxiv/assets/orp/resource/item/60c74d93469df463dff44301/original/docking-studies-of-usnic-acid-and-sodium-usnate-on-sars-co-v-2-main-protease-and-spike-protein-rbd.pdf> [retrieved on 20210914]
- KRISTMUNDSDÓTTIR THÓRDÍS ET AL: "Solubilization of the lichen metabolite (+)-usnic acid for testing in tissue culture", JOURNAL OF PHARMACY AND PHARMACOLOGY : JPP, vol. 54, no. 11, 18 February 2010 (2010-02-18), GB, pages 1447 - 1452, XP055841257, ISSN: 0022-3573, Retrieved from the Internet <URL:https://watermark.silverchair.com/002235702225.pdf?token=AQECAHi208BE49Ooan9kkhW_Ercy7Dm3ZL_9Cf3qfKAc485ysgAAAuEwggLdBgkqhkiG9w0BBwagggLOMIICygIBADCCAsMGCSqGSIb3DQEHATAeBglghkgBZQMEAS4wEQQM4EGpTHOw3IydqaM_AgEQgIIClCozAyRIeB-ptX5jEj0VcikYSjpD5Vu-Q9BRQFxBGwdnnOJX_kkMjEfBhtBzfiOw_fW2TQNZId4IZB67srOnLGl> DOI: 10.1211/002235702225
- GALANTY AGNIESZKA ET AL: "Enantioselective activity of usnic acid: a comprehensive review and future perspectives", PHYTOCHEMISTRY REVIEWS, KLUWER, NL, vol. 18, no. 2, 6 April 2019 (2019-04-06), pages 527 - 548, XP036792805, ISSN: 1568-7767, [retrieved on 20190406], DOI: 10.1007/S11101-019-09605-3

## Description

The present invention relates to a mixture M comprising or, alternatively, consisting of: (a) a usnic acid, and/or (b) a salt thereof, and/or (c) an inclusion compound (ci), or mixtures thereof, said inclusion compound (ci) comprising or, alternatively, consisting of: (i) said (a) an usnic acid and/or said (b) a salt thereof, and (ii) a cyclodextrin, preferably a beta-cyclodextrin, wherein said mixture M is for use as medicament. Furthermore, the present invention relates to a composition, preferably a composition for oral or nasal or otological use (mouth, throat, nose and ear), comprising said mixture M and, optionally, at least one acceptable pharmaceutical or food grade additive and/or excipient; wherein said composition is for use as medicament. Lastly, the present invention relates to said mixture M and said composition comprising said mixture M for use in a method for the treatment of bacterial infections triggered or caused by Gram-positive and/or Gram-negative bacteria, or for use in a method for the treatment of viral infections triggered or caused by viruses, said mixture M and said composition, said composition comprising said mixture M, being capable of inhibiting the spike protein of a virus selected from the group comprising or, alternatively, consisting of coronaviruses, preferably SARS-CoV-2 virus. The Coronavirus disease (COVID-19) is an infection caused by the SARS-CoV-2 virus (SARS=severe acute respiratory syndrome).

The present invention relates to a mixture M and a composition, said composition comprising said mixture M, as an antiviral preferably for oral or nasal or otological (mouth, throat, nose and ear) use as an antiviral agent, preferably for use in the treatment of viral infections of the respiratory system and of symptoms or disorders deriving from or related to said viral infections, preferably viral infections from a SARS-coronavirus (e.g. viral infections from SARS-CoV-2 virus, which causes the COVID-19 disease).

Viral infections of the respiratory tract, as the very name indicates, are infectious diseases caused by viruses that affect the organs of the upper and/or lower respiratory system (nose, mouth, throat, pharynx, larynx, trachea, bronchi and lungs). Preferably, the present invention relates to viral infections caused by at least one virus of the species severe acute respiratory syndrome coronavirus species, abbreviated as SARS-CoV. Said viruses of the SARS-CoV species are positive-strand RNA viruses (group IV of the Baltimore classification), belonging to the genus of *Betacoronavirus.* A virus of the species severe acute respiratory syndrome coronavirus is the virus that caused the 2002-2003 SARS epidemic in China, referred to as SARS-CoV strain. It was first discovered in November 2002 in the Chinese province of Guangdong. From November 1, 2002 to August 31, 2003, the virus infected 8,096 people in about thirty countries, causing 774 deaths, mainly in China, Hong Kong, Taiwan and all of Southeast Asia. Toward the end of 2019, a second virus of the species severe acute respiratory syndrome coronavirus, referred to as SARS-CoV-2 strain or, alternatively, 2019-nCoV, caused a new SARS epidemic in China and in the rest of the world, more commonly known as COVID-19 (*COronaVlrus Disease* 19, also known as SARS-CoV-2 acute respiratory disease or coronavirus disease 2019, also coronavirus syndrome 2019).

Guthappa Roopa: "Docking studies of usnic acid and sodium usnate on SARS CoV-2 main protease and spike protein RBD", 13 July 2020 (2020-07-13) discloses a study showing that usnic acid and salts thereof have binding abilities to main protease and spike protein of SARS-CoV-2.

Said document does not disclose the use of cyclodextrins in combination with usnic acid, nor an inclusion compound comprising at least one cyclodextrin and usnic acid or a salt thereof.

### FIGURES

Figures 1 and 2 are histograms showing the percentage of cell viability after 8 hours and 24 hours, respectively, in the presence of the different concentrations of the usnic acid complex and β-cyclodextrin on the Vero E6 cell line.
Figure 3 is a histogram showing the luminescence value as the multiplicity of infection (MOI) of *Pseudoviruses* varies on the Vero E6 cell line.
Figures 4 A-B, 5 A-B and 6 A-B: are histograms showing the amount of luminescence emitted (A) and the Pseudovirus entry into Vero E6 (B) cells after 1 hour of pre-incubation of *Pseudovirus* containing the SARS-CoV-2 spike protein at MOI 10 with the usnic acid and β-cyclodextrins complex at the concentration of 0.01%, 0.03% and 0.05%, respectively, compared with the results obtained from the *Pseudovirus* without pre-incubation with the virucidal complex (control).
Figure 7 exemplifies a flow chart of a process for the production of usnic acid, according to a possible embodiment.
Figure 8 shows an average distribution of the solid particles of usnic acid according to a possible embodiment.

### SUMMARY OF THE INVENTION

The invention is set out in the appended set of claims.

In a first aspect, the present invention relates to a mixture M (or to a composition comprising said mixture M and additives/excipients) as defined in the present invention for use in a method for the treatment of viral infections, preferably viral infections of the respiratory tract, more preferably viral infections (for example of the respiratory tract) caused by a coronavirus, for example SARS-CoV-2).

In a second aspect, the present invention relates to a method for the treatment of viral infections, preferably viral infections of the respiratory tract, more preferably viral infections (for example of the respiratory tract) caused by a coronavirus (for example SARS-CoV-2), wherein said treatment method provides for administering a therapeutically effective amount of said mixture M (or of said composition comprising said mixture M and additives/excipients) as defined in the present invention, to a subject in need.

In a third aspect, the present description relates to a mixture M (or to a composition comprising said mixture M and additives/excipients) as defined in the present invention for use in a method for the treatment of bacterial infections, preferably bacterial infections of the respiratory tract, more preferably bacterial infections caused by Gram positive and/or Gram-negative bacteria.

In a fourth aspect, the present description relates to a method for the treatment of bacterial infections, preferably bacterial infections of the respiratory tract, more preferably bacterial infections caused by Gram positive and/or Gram-negative bacteria, wherein said treatment method provides for administering a therapeutically effective amount of said mixture M (or of said composition comprising said mixture M and additives/excipients) as defined in the present invention, to a subject in need.

Any references in the description to methods of treatment refer to the compounds, pharmaceutical compositions and medicaments of the present invention for use in a method for treatment of the human (or animal) body by therapy.

### DETAILED DESCRIPTION OF THE INVENTION

Following an extensive research and development activity, the Applicant, addressed and solved the problem relating to the treatment of bacterial infections and viral infections, preferably viral infections of the respiratory tract (upper and lower respiratory tract), in particular, of viral infections of the respiratory tract caused by at least one virus of the species severe acute respiratory syndrome coronavirus (such as SARS-CoV, SARS-CoV-2/2019-nCoV strain - whose disease is known as COVID-19 - or SARS-CoV-like), by providing a mixture M and a composition comprising said mixture M for use as medicament, preferably for oral or nasal use.

The present description relates to a mixture M comprising or, alternatively, consisting of: (a) a usnic acid, and/or (b) a salt thereof, and/or (c) an inclusion compound (ci), or mixtures thereof, said inclusion compound (ci) comprising or, alternatively, consisting of: (i) said (a) an usnic acid and/or said (b) a salt thereof, and (ii) a cyclodextrin, wherein said mixture M is for use as medicament.

Forming an object of the present invention is a composition comprising said mixture M and, optionally, at least one acceptable pharmaceutical or food grade additive and/or excipient; wherein said composition is for use as medicament. According to the invention, said composition is for use in a method for the treatment of a viral infection triggered or caused by at least one coronavirus.

The mixture M and the composition comprising said mixture M have an antibacterial or bacteriostatic and antiviral activity and they are capable of inhibiting a spike protein of a virus selected from the group comprising or, alternatively, consisting of coronaviruses, preferably SARS-CoV-2.

Said mixture M (or a composition comprising said mixture M), is also described for use in a method for the treatment of bacterial infections triggered or caused by Gram-positive and/or Gram-negative bacteria.

Said mixture M (or a composition comprising said mixture M) is for use in a method for the treatment of viral infections triggered or caused by viruses. Preferably, said mixture M (or a composition comprising said mixture M) is capable of inhibiting a spike protein of a virus selected from the group comprising or, alternatively, consisting of coronaviruses, preferably SARS-CoV-2.

The ability/capacity of usnic acid, preferably a combination or association of a dextrorotatory usnic acid of natural origin D(+) and a levorotatory usnic acid of natural origin L(-) 50%-50%, preferably the dextrorotatory form D(+) only, to bind at least one SARS-CoV-2 protease and/or at least one SARS-CoV-2 RBD spike protein, was observed and evaluated. A better binding affinity of natural usnic acid together with the natural usnic acid sodium salt, together with natural usnic acid sodium salt, preferably usnic acid and the salt thereof in the dextrorotatory form D(+) only, in a by weight ratio (usnic acid: usnic acid sodium salt) comprised from 1:10 to 10:1 (for example 1:9, 1:8, 1:7, 1:6, 1:5, 1.4, 1:3, 1:2, 1:1, 2:1, 3:1, 4:1, 5:1, 6:1, 7:1, 8:1, or 9:1), preferably from 1:5 to 5:1, for example from 1:3 to 3:1, from 1:2 to 2:1 or 1:1, with respect to at least one main SARS-CoV-2 protease and/or at least one SARS-CoV-2 RBD spike protein (for example 6M0J) was surprisingly evaluated positively.

The same trend relating to an improved binding affinity was also observed with said (c) an inclusion compound (ci), wherein said inclusion compound (ci) comprises or, alternatively, consists of (i) said (a) an usnic acid and/or said (b) a salt thereof, and (ii) a cyclodextrin, preferably a beta-cyclodextrin. Also in this case, usnic acid, preferably a combination or association of a dextrorotatory usnic acid of natural origin D(+) and a levorotatory usnic acid of natural origin L(-) 50%-50%, is preferably in the dextrorotatory form D(+) only. This usnic acid is capable of binding at least one SARS-CoV-2 protease and/or at least one SARS-CoV-2 RBD spike protein. A better binding affinity of natural usnic acid together with natural usnic acid sodium salt, together with natural usnic acid sodium salt, preferably usnic acid and the salt thereof in the dextrorotatory form D(+) only, in a by weight ratio (usnic acid: usnic acid sodium salt) comprised from 1:10 to 10:1 (for example 1:9, 1:8, 1:7, 1:6, 1:5, 1.4, 1:3, 1:2, 1:1, 2:1, 3:1, 4:1, 5:1, 6:1, 7:1, 8:1, or 9:1), preferably from 1:5 to 5:1, for example from 1:3 to 3:1, from 1:2 to 2:1 or 1:1, with respect to at least one main SARS-CoV-2 protease and/or at least one SARS-CoV-2 RBD spike protein (for example 6M0J) was evaluated positively.

Said better binding affinity of a mixture M or composition of the present invention with respect to at least one main SARS-CoV-2 protease and/or at least one SARS-CoV-2 RBD spike protein (for example 6M0J) was observed in particular when said M mixture comprises or, alternatively, consists of said (ci) inclusion compound comprising or, alternatively, consisting of said (a) a usnic acid and (ii) a cyclodextrin: alpha, beta and/or gamma cyclodextrin, preferably a beta-cyclodextrin, preferably wherein the by weight ratio between (a) usnic acid and (ii) a cyclodextrin, preferably a beta-cyclodextrin [(a):(ii)], is comprised from 1:5 to 5:1, preferably from 1:3 to 3:1, even more preferably from 1:2 to 2:1, for example about 1:1, or 1:2 (usnic acid:cyclodextrin).

In an embodiment, said mixture M (or a composition comprising said mixture M) comprises or, alternatively, consists of said (a) a usnic acid of natural origin which is preferably in racemic form 50% (+) and 50% (-), or in dextrorotatory form D(+) only. Said (b) is, preferably, a usnic acid salt of natural origin. Said salt is an alkali metal or alkaline-earth metal salt; preferably said usnic acid salt of natural origin is a usnic acid sodium salt.

In an embodiment, the mixture M (or a composition comprising said mixture M) comprises or, alternatively, consists of said (a) an usnic acid of natural origin; preferably said mixture M (or a composition comprising said mixture M) is for oral or nasal or otological (mouth, throat, nose and ear) use in a method for the treatment of bacterial or viral infections which affect human beings and animals, preferably it is for oral use in the human and veterinary field. The mixture M and the composition comprising said mixture M, subject of the present invention, are not used in the treatment of dental caries or in the preventive treatment of cariogenic dental plaques, nor, much less, against the bacterium *Streptococcus mutans* (Gram-positive) which is one of the main pathogens in causing caries and cariogenic lesions.

In another embodiment, the mixture M (or a composition comprising said mixture M) comprises or, alternatively, consists of said (a) an usnic acid of natural origin; preferably said mixture M (or a composition comprising said mixture M) is for use in a method for the treatment of a viral infection of the respiratory system and of symptoms and/or disorders deriving from or related to said viral infection; preferably viral infections of the upper respiratory tract and/or of the lower respiratory tract. Said viral infection is triggered or caused by a virus of the family *Coronaviridae,* subfamily: *Coronavirinae,* genus: *Betacoronavirus,* species: severe acute respiratory syndrome coronavirus, selected from the strains: severe acute respiratory syndrome coronavirus (SARS-CoV), severe acute respiratory syndrome coronavirus-2 (SARS-CoV-2 or 2019-nCoV) and responsible for COVID-19 disease, and severe acute respiratory syndrome coronavirus-like (SARS-CoV-like or SL-CoV); preferably SARS-CoV-2. Said mixture M is capable of binding with at least one SARS-CoV-2 protease and/or at least one spike protein; preferably with the SARS-CoV-2 RBD spike protein (for example 6M0J).

In an embodiment, the mixture M (or a composition comprising said mixture M) is for use in a method for the treatment of symptoms and/or disorders, said symptoms and/or disorders (deriving from or related to said viral infection of the respiratory system) are selected from: severe acute respiratory syndrome (SARS), respiratory complications, asthma, chronic obstructive pulmonary disease (COPD), bronchitis, emphysema, cystic fibrosis, cough, pertussis, pneumonia, pleuritis, bronchiolitis, cold, sinusitis, rhinitis, tracheitis, pharyngitis, laryngitis, acute laryngotracheobronchitis, epiglottitis, bronchiectasis, difficulty breathing, dyspnoea, breathlessness, shortness of breath, fever, fatigue, muscle aches, muscle pain, nasal congestion, runny nose, sore throat, gastrointestinal symptoms, nausea, diarrhoea, renal insufficiency, loss of appetite, general feeling of malaise.

In an embodiment, the mixture M (or a composition comprising said mixture M) comprises or, alternatively, consists of (a) an usnic acid of natural origin which is present as a combination or association (C/A) between a dextrorotatory natural usnic acid D(+) and a levorotatory natural usnic acid L(-); preferably the dextrorotatory form D(+) is comprised from 0.1% to 99.9% by weight (for example (1%, 10%, 20%, 30%, 40%, 50%, 60%, 70%, 80% or 90%), with respect to the total weight of the combination or association (C/A), and/or the levorotatory form L(-) is comprised from 99.9% to 0.1% by weight (for example 90%, 80%, 70%, 60%, 50%, 40%, 30%, 20%, or 10%), with respect to the total weight of the combination or association. Preferably, said (a) an usnic acid of natural origin is in racemic form 50% (+) and 50% (-), or in dextrorotatory form D(+) (100%).

In an embodiment, the mixture M (or a composition comprising said mixture M) comprises or, alternatively, consists of said (b) a usnic acid salt of natural origin, which is present as an alkali metal or alkaline-earth metal salt; preferably said usnic acid salt of natural origin is a usnic acid sodium salt. Preferably, said (b) a natural usnic acid salt is in racemic form, or dextrorotatory form D(+); preferably said salt may be present in dextrorotatory form D(+) in an amount by weight comprised from 0.1% to 99.9% by weight (for example (1%, 10%, 20%, 30%, 40%, 50%, 60%, 70%, 80% or 90%), with respect to the total weight of the combination or association (C/A), and/or in levorotatory form L(-) in an amount comprised from 99.9% to 0.1% by weight (for example 90%, 80%, 70%, 60%, 50%, 40%, 30%, 20%, or 10%), with respect to the total weight of the combination or association (C/A).

Preferably, said (a) an usnic acid of natural origin and said (b), a salt thereof, are present in the mixture M (or a composition comprising said mixture M) in a by weight ratio comprised from 1:10 to 10:1 (for example 1:9, 1:8, 1:7, 1:6, 1:5, 1.4, 1:3, 1:2, 1:1, 2:1, 3:1, 4:1, 5:1, 6:1, 7:1, 8:1, or 9:1), preferably from 1:5 to 5:1, even more preferably from 1:3 to 3:1, for example 1:1.

Preferably, said (a) an usnic acid of natural origin and/or said (b), a salt thereof, are present in the mixture M (or a composition comprising said mixture M) in an amount by weight comprised from 1:10 to 9:10 (for example 1.5:10, 2:10, 2.5:10, 3:10, 3.5:10, 4:10, 4.5:10, 5:10, 5.5:10, 6:10, 6.5:10, 7:10, 7.5:10, 8:10, or 8.5:10), preferably from 1:10 to 6:10 or from 4:10 to 8:10, with respect to the total weight of the mixture M (or a composition comprising said mixture M), wherein the weight of said mixture M is 10.

The chemical name of usnic acid (D(+) and/or L(-)) is 2,6-diacetyl-7,9-dihydroxy-8,9b-dimethyl-1,3(2H,9bH)-dibenzofurandione or 2,6-diacetyl-7,9-dihydroxy-8,9b-dimethyldibenzo[b,d]furan-1,3(2H,9bH)-dione.

Preferably, the usnic acid which can be used in the present invention may be represented by the compound: (+)-usnic acid 2,6-diacetyl-7,9-dihydroxy-8,9b-dimethyldibenzo[b,d]furan-1,3(2H,9bH)-dione; (+)-usnic acid from *Usnea*; for example CAS of (+) usnic acid: 7562-61-0, EC of (+) usnic acid: 231-456-0; preferably, the usnic acid sodium salt which can be used in the present invention may be represented by the compound: monosodium salt of 2,6-diacetyl-7,9-dihydrocy-8,9b-dimethyldibenzo-1,3(2H,9bH)-dione; CAS N°: 34769-44-3, EC: 252-204-6; preferably the purity of said (a) an usnic acid and/or of said (b) an usnic acid salt is comprised from 95% to 99.9%, preferably from 96% to 99.5%, even more preferably from 97% to 98% or 98%, for example 98% (% weight/weight).

Described is a mixture M (or a composition comprising said mixture M), wherein said mixture M (or a composition comprising said mixture M) comprises or, alternatively, consists of (c) an inclusion compound (CI) comprising or, alternatively, consisting of: (i) said (a) an usnic acid and/or said (b) a salt thereof, and (ii) a cyclodextrin: alpha, beta and/or gamma cyclodextrin, preferably a beta-cyclodextrin, said mixture M (or a composition comprising said mixture M) being for use as medicament; preferably for use in a method for the treatment of bacterial infections triggered or caused by Gram-positive and/or Gram-negative bacteria, or for use in a method for the treatment of viral infections triggered or caused by viruses. Preferably, said mixture M (or a composition comprising said mixture M) comprising said inclusion compound (ci) is capable of inhibiting the spike protein of a virus selected from the group comprising or, alternatively, consisting of coronaviruses; more preferably said inclusion compound (ci) is capable of binding at least one SARS-CoV-2 protease and/or at least one SARS-CoV-2 RBD spike protein (for example 6M0J).

In the context of the present description, the expression "inclusion compound" is used to indicate a chemical structure of the type similar to a chemical complex in which a chemical compound (host) may have cavities (for example, one or two or three cavities, preferably one) of certain dimensions, identical or different from each other, in which there can be allocated or positioned or established molecules (for example one or two or three molecules, preferably one) having dimensions similar to those of the respective cavities, of a second chemical compound (guest), where the host and the guest are non-covalently bound, generally by means of intermolecular forces such as van der Waals forces, or electrostatic bonds or interactions between the host and the guest charged with a charge of opposite sign.

The "inclusion compound" may, for example, be obtained by contacting (a) and/or (b) with (ii) a cyclodextrin, in given weight ratios, preferably in the form of powders or granules or flakes, for a given mixing time in an equipment which allows to carry out a mechanical mixing, such as for example a ball mill, of (a) and/or (b) with (ii) a cyclodextrin to obtain (ci). The cyclodextrins are natural cyclic oligosaccharides formed by 6, 7 or 8 D-(+) glucopyranose monomers joined together with a α,1-4 glucosidic bond and closed in a ring which have cavities (for example, one or two cavities) of given dimensions, identical or different from each other. In the inclusion compound (ci) of the present invention, the cyclodextrins (ii) form a molecular cage which delimits a lipophilic cavity capable of hosting, thanks to the energy provided by a mechanical impact and/or mixing force for example provided with a ball mill, said (i) such as usnic acid (a) or a salt thereof (b) or mixtures thereof.

Preferably, said cyclodextrins (ii) used in the inclusion compound (ci) are selected from the group comprising or, alternatively, consisting of α-cyclodextrins, β-cyclodextrins, γ-cyclodextrins and mixtures thereof. More preferably, said cyclodextrins (ii) are beta-cyclodextrins.

Preferably, said at least one cyclodextrin (ii), comprised in the inclusion compound (ci) together with (a) and/or (b), is a beta-cyclodextrin; preferably it is a (2-hydroxypropyl)-β-cyclodextrin; said at least one cyclodextrin (ii) is present in said inclusion compound (ci) in a by weight ratio comprised from 3:1 to 1:3, preferably comprised from 2:1 to 1:2, more preferably comprised from 1.5:1 to 1:1.5, even more preferably being 1:1, with respect to said (a) and/or said (b) (*i.e.* [(ii):(a)] or [(ii):[(a)+)b)]]). An example of (2-hydroxypropyl)-β-cyclodextrin which can be used in the context of the present invention is (2-hydroxypropyl)-β-cyclodextrin having an average molecular weight comprised from about 1350-1380 to 1460-1480 and/or a CAS No. 128446-35-5.

In a preferred embodiment, said mixture M (or a composition comprising said mixture M) consists of said (c) inclusion compound (ci) comprising or, alternatively, consisting of said (a) usnic acid (or a salt thereof) and (ii) a beta-cyclodextrin, preferably a (2-hydroxypropyl)-β-cyclodextrin.

In a preferred embodiment, said mixture M (or a composition comprising said mixture M) consists of said (c) inclusion compound (ci) comprising or, alternatively, consisting of: said (a) usnic acid (or a salt thereof) and said (ii) beta-cyclodextrin (preferably (2-hydroxypropyl)-β-cyclodextrin), wherein said (a) usnic acid and said (ii) beta-cyclodextrin are present in a [(a):(ii)] by weight ratio comprised from 1:5 to 5:1, preferably from 1:3 to 3:1, even more preferably from 1:2 to 2:1, for example about 1:1, or 1:2 (usnic acid:cyclodextrin).

According to an example of said inclusion compound (ci), comprising or, alternatively, consisting of (a) usnic acid (or a salt thereof) and (ii) beta-cyclodextrin (preferably (2-hydroxypropyl)-β-cyclodextrin) in a [(a): (ii)] by weight ratio of about 1:1 or 1:2, wherein preferably said (a) usnic acid is D(+)usnic acid as substantially pure enantiomer and said (ii) β-cyclodextrin is beta-cyclodextrin=(2-hydroxypropyl)-β-cyclodextrin.

Preferably, said mixture M (or a composition comprising said mixture M), according to any one of the described embodiments, may be in the solid or semi-solid state, in dispersed or suspended form, in the form of a gel, or in the liquid state; preferably said mixture M (or a composition comprising said mixture M) may be in the form of flakes, granules, powders, pellets, or it may be an aqueous or hydroalcoholic solution or in organic solvents, such as for example an aqueous spray solution (which can be sprayed (sprayable). Preferably, said (a) an usnic acid of natural origin and/or said (b) a salt thereof are in powdered solid form with an average particle size comprised from 0.1 or 1 microns to 100 microns (for example 0.5 µm, 5 µm, 10 µm, 20 µm, 30 µm, 40 µm, 50 µm, 60 µm, 70 µm, 80 µm, or 90 µm), preferably from 5 microns to 50 microns, even more preferably from 10 microns to 20 microns (microns = µm; 1 µm=10⁻⁶ m; the dimensions of the cells are in the order of magnitude comprised from about 10 µm to about 50 µm).

Said mixture M (or a composition comprising said mixture is for use in the human or veterinary field as an antibacterial agent, antibacterial proliferative, bacteriostatic agent, microbicidal agent, anti-mould agent, anti-yeast agent (example *Candida*), anti-fungal or antimycotic agent (example *Saccharomycetes*)*,* preferably it is for use against Gram-positive and/or Gram-negative bacteria, such as for example those having the scientific name *Klebsiella, Enterobatteriacee, Enterobacter, Pseudonomas* and *Escherichia.*

According to the present description, said mixture M (or a composition comprising said mixture M), is also for use as antibacterial or bacteriostatic agent both for Gram-Positive bacteria and Gram-negative bacteria; wherein said bacteria are preferably selected from the group comprising or, alternatively, consisting of: *Escherichia Coli, Klebsiella, Acinetobacter baumannii, Staphylococcus aureus,* methicillin resistant *Staphylococcus aureus* (MRSA), *Enterococcus,* Vancomycin-resistant *Enterococcus spp.* (VRE), *Actinobacter, Actinobacter spp., Clostridium difficile,* and combinations thereof.

Advantageously, the usnic acid and/or a salt thereof which can be used in the context of the present invention (for example in the inclusion compound (ci)) is extracted from lichens; preferably said lichens are selected from the group comprising or, alternatively, consisting of *Usnea, Cladonia, Hypotrachyna, Lecanora, Ramalina, Evernia, Parmelia, Alectoria* and combinations thereof, more preferably said lichens are selected from Usnea, even more preferably from *Usnea longissima* Ach..

In the inclusion compound (ci) of the present invention, said cyclodextrins (ii) comprise or, alternatively, consist of beta-cyclodextrins, preferably said cyclodextrin (ii) is (2-hydroxypropyl)-β-cyclodextrin, more preferably in a (ii):(i) by weight ratio equal to about 1:1.

Said inclusion compound (ci) comprises, together with said (ii) cyclodextrin, solid particles of said (a) an usnic acid, preferably D(+)-usnic acid as pure enantiomer (or as substantially pure enantiomer), or a salt thereof, wherein said solid particles have an average particle size distribution comprised from 0.01 µm to 50 µm (for example 0.1 µm, 0.5 µm, 1 µm, 10 µm, 20 µm, 30 µm, or 40 µm), preferably comprised from 0.1 µm to 30 µm, more preferably comprised from 0.15 µm to 20 µm, even more preferably comprised from 0.2 µm to 15 µm.

Said average particle size distribution of said (a) an usnic acid may be determined and measured for example using a laser diffraction method, according to the GB/T 19077-2016 standard, for example using a Malvern Mastersizer 3000 instrument. Said standard is meant in the version valid at the actual of the present patent application.

Preferably, said solid particles of said (a) an usnic acid have an average particle size distribution such that D10 = 0.236 µm, D50 = 1.570 µm, and D90 = 31.800 µm.

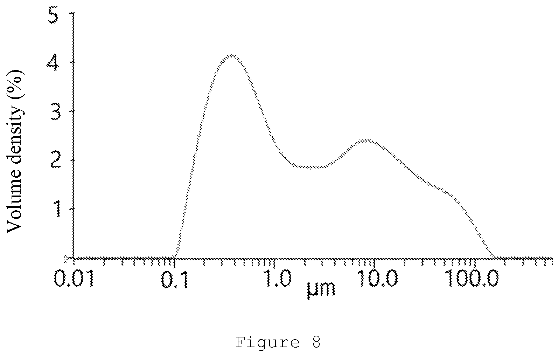

In an embodiment of the present invention, said solid particles have a distribution according to the chart of figure 8.

Forming an object of the present invention is a composition comprising said mixture M and, optionally, at least one acceptable pharmaceutical or food grade additive and/or excipient; wherein said composition is for use as medicament.

In an embodiment, said composition is a pharmaceutical composition, a medical device composition according to Regulation (EU) 2017/745, a composition for a Food for Special Medical Purposes (FSMP), a composition for *Novel Food* according to Regulation (EU) 2015/2283, or a composition for foodstuffs or dietary supplements.

In an embodiment, said composition is for internal, external, parenteral, topical, dermatological use; or it is for use on tissues, dermis or mucosa; or it is for oral, nasal, ocular use; or it is for use in the ear canal, throat, trachea, oesophagus, eye, nose or ear; or it is for use on the oral mucosa, nasal mucosa, ocular mucosa or of the ear canal; or it for use in the stomach, bowel, gastro-intestinal use; or it is for vaginal or rectal use, both in the human and veterinary field fort humans and animals.

Preferably, said composition is in solid form selected from: tablets, chewable tablets, mouth dissolvable tablets, granules, powder, flakes, soluble or water-soluble powder or granules, mouth dissolvable powders or granules, capsules; or in liquid form selected from: solutions, suspensions, dispersions, emulsions, liquid which can be dispensed in form of spray, oral spray or nasal spray, dry powder for oral inhalation, aerosol, oral aerosol or nasal aerosol, syrups; or in semiliquid or semisolid form selected from: soft-gel, gel, ointment, cream, cream for topical (dermal), oral, ocular or nasal use.

In an embodiment, the composition of the present invention is formulated by using at least one acceptable pharmaceutical or food grade additive and/or excipient which is selected from: collagen, chondroitin, hyaluronic acid or a salt thereof, such as sodium hyaluronate, vitamins, amino acids, antioxidants, silica, cellulose, carboxy cellulose, carboxymethyl cellulose (CMC), carboxyethyl cellulose, carboxypropyl cellulose, hydroxypropyl cellulose (HPC), hydroxypropyl methylcellulose (HPMC), hydroxymethyl cellulose, hydroxyethyl cellulose, hydroxypropyl cellulose, poloxamers, fibres, inulin, maltodextrin, fats, waxes, oils, animal oils, vegetable oils, vaseline, carbonate, bicarbonate, ammonium salts, sodium chloride, clays, kaolin, antioxidants such as for example glutathione, quercetin, coenzyme Q10 or vitamins, in particular vitamins of group C or E, such as for example tocopherols and tocotrienols, tocotrienols of plant origin such as for example tocotrienols from rice, preservatives such as for example sodium and potassium benzoate, or sodium or potassium sorbate, mineral salts, sodium chloride, and pH buffers such as for example citric acid and a soluble salt thereof, for example citric acid monohydrate.

A preferred embodiment of the composition, subject of the present invention, aims at providing an aqueous liquid solution which is administered using an atomiser or spray can. Said composition is formulated using food or pharmaceutical grade additives and/or excipients which allow, once the liquid composition, for example at a room temperature of about 20°C or 5°C, is applied through the nasal route to a subject with a body temperature for example comprised from 36°C to 40°C, to bring the liquid aqueous composition to room temperature to take a consistency of a gel. For example, they may be used in an aqueous composition to be atomised, combined or associated with (a) and/or (b) and (ii) a cyclodextrin, hydroxypropyl methylcellulose (HPMC) and tocotrienols, preferably tocotrienols from rice. Preferably, tocotrienols from rice *(Oryza sativa* L.) may be in the form of a water-dispersible powder having for example a tocotrienol content comprised from 5% to 75%, preferably from 15% to 60%, for example 25%, or 30%, or 40%, or 50% by weight (product under the trade name TheraPrimE^{®} -BGG). The water-dispersible powder may contain, together with the tocotrienols, also preferably maltodextrins (9050-36-6), starch and silicon dioxide SiO2 (7631-86-9). Preferably, the water-dispersible powder may contain a rice bran oil extract from 30% to 60%, modified starch from 25% to 45%, maltodextrin from 20% to 40% and SiO2 from 0.2 to 1.5%. Preferably, a water-dispersible powder containing a mixture of tocotrienols and tocopherols in a total amount by weight comprised from 15% to 60%, for example 25%, or 30%, or 40%, or 50% may be used; preferably in the case of a total content in the mixture of 25% by weight, there may be for example an amount of about 10% by weight of tocotrienols and an amount of about 15% by weight of tocopherols (amount measured by means of HPLC), while in the case of a total content in the mixture of 30% by weight there may be about 15% by weight of tocotrienols and about 15% by weight of tocopherols, the remaining part at 100% may be starch, maltodextrins, SiO2 and other. The usnic acid D(+) of the present invention may preferably have a melting point (DSC) comprised from 190°C to 210°C, preferably from 192°C to 198°C, a molecular rotation comprised from 480° to 540°, preferably from 490° to 520°; a PSD value: D50 comprised from 1 micron to 15 microns, preferably from 2 microns to 10 microns, for example 5 microns. Preferably, said usnic acid has a low content of pathogens such as *Staffilococco Aureus* MIC less than or equal to 500 ppm and *Escherichia Coli* MIC less than or equal to 1000 ppm.

A preferred embodiment of the composition, subject of the present invention, aims at providing an aqueous liquid solution as reported in the table below:

| Ingredients | Compositions (from x% to y%) | | | |
|---|---|---|---|---|
| Water | 94-99 | 95-98 | 96-97.8 | Aqueous liquid solutions at 20°C |
| CMC, or HPC, or HPMC | 0.25-2.5 | 0.5-1.5 | 0.65-0.95 | |
| Usnic acid, or a sodium salt | 0.0001-0.5 | 0.0005-0.05 | 0.001-0.0075 | |
| Beta-cyclodextrin | 0.005-0.5 | 0.0075-0.25 | 0.01-0.015 | |
| NaCl | 0.1-2 | 0.3-1.5 | 0.5-0.9 | |
| Sodium Benzoate | 0.001-1 | 0.01-0.75 | 0.05-0.1 | |
| Potassium Sorbate | 0.001-1 | 0.01-0.75 | 0.05-0.1 | |
| Tocotrienols, or mixture of tocotrienols and tocopherols | 0.001-1 | 0.01-0.75 | 0.05-0.1 | |
| Citric acid | 0.001-0.1 | 0.01-0.005 | 0.015-0.02 | |
| Total weight (w/w) | 100 | | | |
| Density 1g/cc | | | | |

In an embodiment, the composition of the present invention - for example like those reported in the table above - is in the form of an aqueous solution and it comprises or, alternatively, consists of:
- water, preferably demineralised water, in an amount by weight comprised from 90% to 99.5%, preferably from 94% to 99%;
- (a) usnic acid and/or (b) a usnic acid salt and/or (c) an inclusion compound (ci), or mixtures thereof, preferably in the form D(+), in an amount by weight comprised from 0.0001% to 0.5%, preferably from 0.01 to 0.25%;
- a polymer selected from the group comprising or, alternatively, consisting of: cellulose, carboxy cellulose, carboxymethyl cellulose (CMC), carboxyethyl cellulose, carboxypropyl cellulose, hydroxypropyl cellulose (HPC), hydroxypropyl methylcellulose (HPMC), hydroxymethyl cellulose, hydroxyethyl cellulose, hydroxypropyl cellulose, poloxamers, preferably hydroxypropyl cellulose (HPMC), in an amount by weight comprised from 0.25% to 2.5%, preferably from 0.5% to 1.5%;
- a compound with antioxidant activity selected from the group comprising or, alternatively, consisting of: glutathione, quercetin, coenzyme Q10, vitamins, in particular the vitamins of group C or E, such as for example tocopherols and tocotrienols, preferably tocotrienols of plant origin such as for example tocotrienols from rice;
- preferably mineral salts, preservative agents and pH buffers or stabilisers commonly used in the industry.

Said composition in the form of an aqueous solution may preferably have a viscosity value at 20°C (mPa•second) of about 240 +/- 40; a pH value of about 5 +/- 0.2, a density value g/cc of about 1.01 +/- 0.1 and a microbiological load (total load, yeasts, moulds, 2 pathogens (*Staphilococco Aureus* and *Pseudomonas))* of the type: Total load <= 100 CFUs, yeasts <= 100 CFUs, Moulds <= 10 CFUs and absence of pathogens. Preferably, the composition of the present invention - for example like those reported in the table above - may be prepared using a preparation method comprising or, alternatively, consisting of the following steps:
1) a step for drying the powders, preferably the active ingredients (a) and/or b and/or (c) the inclusion compound (ci) and the polymers CMC, or HPC, or HPMC, at a temperature preferably comprised from 90°C to 150°C, even more preferably from 100°C to 110°C;
2) a step for adding the powders according to step 1) in a water volume, for example 100 litres of demineralised water, previously heated to a temperature comprised from 50°C to 90°C, preferably from 60°C to 80°C +/-2°C, to obtain a solution;
3) a step for mixing said solution according to step 2), preferably by using a magnetic mixer, for example for a period of time comprised from 30 minutes to 240 minutes, preferably from 60 minutes to 120 minutes, to obtain a final aqueous solution; and optionally
4) a step for controlling and adjusting the pH value to a value comprised from 5±0.1 to 7±0.1, preferably from 5.5±0.1 to 6.5±0.1, to obtain a final solution (subject of the present invention). Preferably, said final solution is ready to be packaged, for example in an atomiser container to obtain a spray solution which, in contact with the body temperature of a subject, takes a consistency similar to a gel or cream, in particular if said solution is applied through the nasal route.

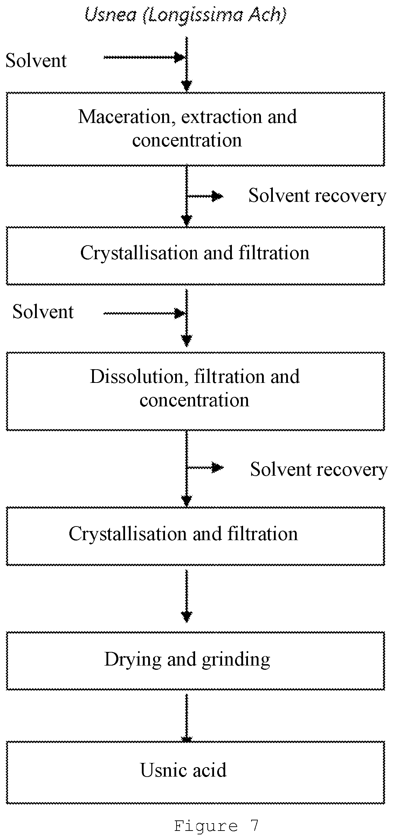

The attached figure 7 schematically shows a flow chart of a process for obtaining the usnic acid of natural origin, preferably D(+)-usnic acid. The usnic acid in dried form is obtained after the following steps:
(a.1) maceration and extraction from a plant material selected from a lichen preferably selected from the group comprising or, alternatively, consisting of *Usnea, Cladonia, Hypotrachyna, Lecanora, Ramalina, Evernia, Parmelia, Alectoria* and combinations thereof, more preferably of *Usnea,* even more preferably of *Usnea Longissima* Ach., with an organic solvent, preferably a solvent selected from the group comprising or, alternatively, consisting of benzene, hexane, acetone, chloroform, trichloroethylene, or an alcoholic solvent, even more preferably ethanol, to obtain an extraction solution, and concentration of said extraction solution to obtain a concentrated extraction solution and a residual solvent;
(a.2) crystallisation and filtration of the concentrated extraction solution, obtained from step (a.1), to obtain a crystallised and filtered extraction product;
(a.3) dissolution, filtration and concentration of the crystallised and filtered extraction product, obtained from step (a.2), to obtain a concentrated extract and a residual solvent;
(a.4) crystallisation, filtration, and subsequently drying and grinding, of the concentrated extract, obtained from step (a.3), to obtain a dry ground extract of usnic acid, preferably having a titre comprised from 80% to 99.9%, more preferably comprised from 90% to 99.5%, even more preferably comprised from 95% to 98% (% weight/weight or volume/volume).

The maceration and extraction of step (a.1) is preferably carried out in an extraction tank, preferably made of stainless steel, provided with stirring and heating means. Preferably, in the maceration and extraction of step (a.1) a [weight of plant material]:[volume of organic solvent] ratio comprised from 10:1 to 1:50, preferably comprised from 5:1 to 1:40, even more preferably comprised from 1:1 to 1:35, is used. The maceration and extraction of step (a.1) are preferably carried out at ambient pressure, and at a temperature comprised from 10°C to 80°C, preferably comprised from 20°C to 70°C, even more preferably comprised from 25°C to 60°C.

In step (a.1) the thallus (sprout or scion) of *Usnea* (*Longissima Ach.*) is preferably used together with the ethyl acetate solvent, for example in an amount of, for example, 350 Kg of plant part and 2600 litres of solvent. The maceration is preferably carried out at a temperature of about 25°C and pressure of 1 atmosphere for a period of time comprised from 2 hours to 10 hours, preferably from 4 hours to 8 hours, for example from 5 hours to 6 hours. The maceration can be carried out in a reactor provided with means for stirring, heating and recycling liquids. For example, maceration is carried out by continuously recirculating the distillate (solvent) on the plant part. The extraction, carried out as a single step, is preferably carried out at a temperature of about 25°C and pressure of 1 atmosphere. The concentration of the extraction solvent used, containing the usnic acid extracted from the plant part, is preferably carried out considering the boiling temperature of ethyl acetate which is about 77.1°C, possibly also acting on the extraction pressure. A dense concentrated liquid and solvent recovery, almost completely, are obtained.

In the crystallisation and filtration of step (a.2), subsequent to step (a.1), to obtain the crystallised and filtered extraction product, an organic solvent selected from the group comprising or, alternatively, consisting of benzene, hexane, acetone, chloroform, trichloroethylene, or an alcoholic solvent, even more preferably ethanol, is preferably used. In the crystallisation and filtration of step (a.2) a [concentrated extraction solution]:[organic solvent] by volume ratio comprised from 10:1 to 1:40, preferably comprised from 5:1 to 1:30, even more preferably comprised from 1:1 to 1:20, is preferably used. In the crystallisation of step (a.2), the concentrated extraction solution obtained from step (a.1) is preferably cooled to facilitate crystallisation, more preferably at a temperature comprised from 1°C to 20°C at ambient pressure, even more preferably comprised from 5°C to 15°C at ambient pressure. At the end of step (a.2) a crystal material with a purity of at least 80%, from 85% to 90% and in an amount of about 20 Kg, if starting from about 350 Kg of plant part is obtained.

In step (a.3), subsequent to step (a.2), the crystallised and filtered extraction product obtained from step (a.2) is dissolved, filtered and concentrated to obtain a concentrated extract and the residual solvent. In step (a.3) an organic solvent, more preferably selected from the group comprising, or alternatively, consisting of benzene, hexane, acetone, chloroform, trichloroethylene, or an alcoholic solvent, even more preferably ethanol, is preferably used. In the dissolution of step (a.3) a [crystallised and filtered extraction product weight]:[organic solvent volume] ratio comprised from 10:1 to 1:40, preferably comprised from 5:1 to 1:30, even more preferably comprised from 1:1 to 1:20, is preferably used. In step (a.3) they are dissolved in chloroform 2x20 Kg to obtain 20 Kg with a minimum purity of 98% of usnic acid.

In step (a.4) subsequent to step (a.3), the concentrated extract obtained from step (a.3) is crystallised, filtered, and subsequently dried and ground, to obtain the dry ground extract of usnic acid. In crystallisation of step (a.4) an organic solvent, more preferably selected from the group comprising, or alternatively, consisting of benzene, hexane, acetone, chloroform, trichloroethylene, or an alcoholic solvent, even more preferably ethanol, is preferably used. In the crystallisation of step (a.4) a [concentrated extract weight]: [organic solvent volume] ratio comprised from 10:1 to 1:40, preferably comprised from 5:1 to 1:30, even more preferably comprised from 1:1 to 1:20, is preferably used. In the crystallisation of step (a.4), the concentrated extract obtained from step (a.3) is preferably cooled to facilitate crystallisation, more preferably at a temperature comprised from 1°C to 20°C at ambient pressure, even more preferably comprised from 5°C to 15°C at ambient pressure. The drying of step (a.4) is preferably carried out up to a residual solvent content comprised from 0.5% to 10% by weight, preferably comprised from 1% to 5% by weight, even more preferably comprised from 1.5% to 3% by weight, with respect to the total weight of the dry extract of usnic acid. Preferably, the grinding of step (a.4) is carried out by means of a mill, more preferably a rotary ball mill. The drying of the filtered and crystallised solid obtained from step (a.4) is complete, for example, when a residual solvent content equal to about 2%-5% by weight, with respect to the initial weight, remains. A plate dryer (without pressure vacuum) is used at a temperature preferably of about 95°C-99°C with air circulation. The ground solid has, for example, an average particle size distribution comprised from 20 mesh to 40 mesh and it contains 98% by weight usnic acid (HPLC with Sigma Aldrich method). Starting from 2x350 Kg of plant part at the beginning of the process (starting material), about 3%-4% of yield of material (dry solid) can be obtained at the end of the process, which is equivalent to about 14 Kg-28 Kg of usnic acid with a content of 98% by weight (13.72 Kg-27.44 Kg). The obtained usnic acid is in the D(+) form 99.9% pure usnic acid, or as racemate.

After obtaining the dry ground extract of usnic acid (step (a.4)) as racemic mixture, said dry extract is selectively complexed with cyclodextrins, preferably beta-cyclodextrins, to obtain said inclusion compound (ci). Preferably, said selective complexation is obtained by means of a co-precipitation of usnic acid, or salt thereof, or mixtures thereof (i) and cyclodextrins (ii). The inclusion compound (ci) is preferably obtained by means of a co-precipitation of usnic acid (preferably D(+)-usnic acid), or salt thereof, or mixtures thereof (i) and cyclodextrins (ii), preferably beta-cyclodextrins. For example, the cyclodextrins (ii) are initially dissolved in water or other suitable aqueous solvent, and subsequently the dry ground extract of usnic acid of step (a.4) is added, while the aqueous solution containing the cyclodextrins (ii) is kept under stirring. In the presence of a sufficiently high concentration of cyclodextrins (ii) in solution, precipitation of the inclusion compound (ci) will begin as the complexation reaction of usnic acid, or salt thereof, or mixtures thereof (i) by cyclodextrins (ii) progressively proceeds. Preferably, the solution containing the inclusion compounds (ci) may have to be cooled to a temperature comprised from 1°C to 18°C, preferably under stirring, in order to initiate precipitation. The inclusion compounds (ci) may be collected through decantation, centrifugation or filtration. The inclusion compound (ci) is preferably a water-soluble clathrate or a water-suspendable clathrate, wherein said usnic acid (as pure enantiomer D(+) or as racemic), or salt thereof, or mixtures thereof (i) is hosted in a cavity of said clathrate, once said D-usnic acid, or salt thereof, or mixtures thereof (i) is contacted with said cyclodextrins (ii).

The present disclosure describes:
It is disclosed a mixture M for use in a method for the treatment of a viral infection triggered or caused by at least one virus,
wherein said mixture M comprises or, alternatively, consists of:
   - (a) an usnic acid, and/or
   - (b) a salt thereof, and/or
   - (c) an inclusion compound (ci), or mixtures thereof, said inclusion compound (ci) comprising or, alternatively, consisting of:
      - (i) said (a) an usnic acid and/or said (b) a salt thereof, and
      - (ii) at least one cyclodextrin.

Preferably, said mixture M is for use in a method for the treatment of viral infections triggered or caused by a coronavirus, preferably a SARS-CoV-2 virus; more preferably said mixture M is capable of inhibiting the spike protein of a coronavirus, preferably of a SARS-CoV-2 virus.

According to the invention, said mixture M comprises or, alternatively, consists of said (c) inclusion compound (ci),
wherein said inclusion compound (ci) comprises or, alternatively, consists of:
- (i) said (a) usnic acid and/or said (b) salt thereof, and
- (ii) at least one cyclodextrin.

Preferably, said (ii) at least one cyclodextrin comprises or, alternatively, consists of at least one beta-cyclodextrin.

Preferably, said (ii) at least one beta-cyclodextrin comprises or, alternatively, consists of a (2-hydroxypropyl)-beta-cyclodextrin.

Preferably, in said inclusion compound (ci) said (ii) at least one cyclodextrin, preferably at least one beta-cyclodextrin, and said (i) usnic acid and/or a salt thereof are in a [(ii):(i)] by weight ratio comprised from 3:1 to 1:3, preferably comprised from 2:1 to 1:2, more preferably comprised from 1.5:1 to 1:1.5, even more preferably being 1:1.

Preferably, said (a) an usnic acid is of natural origin; preferably wherein said mixture M is for oral use in a method for the treatment of viral infections which affect humans and animals; more preferably for oral use in the human field.

Preferably, said mixture M is for use in a method for the treatment of a viral infection of the respiratory system and of symptoms and/or disorders deriving from or relating to said viral infection; preferably viral infections of the upper respiratory tract and/or of the lower respiratory tract.

Preferably, said viral infection triggered or caused by a virus of the family *Coronaviridae,* subfamily: *Coronavirinae,* genus: *Betacoronavirus,* species: severe acute respiratory syndrome coronavirus, selected from strains: severe acute respiratory syndrome coronavirus (SARS-CoV), severe acute respiratory syndrome coronavirus-2 (SARS-CoV-2 or 2019-nCoV) and responsible for COVID-19 disease, and severe acute respiratory syndrome coronavirus-like SARS-CoV-like or SL-CoV); preferably SARS-CoV-2.

Preferably, said mixture M is capable of binding with at least one SARS-CoV-2 protease and/or with a SARS-CoV-2 spike protein; preferably with the SARS-CoV-2 RBD spike protein.

Preferably, said symptoms and/or disorders deriving from or related to said viral infection of the respiratory system are selected from: severe acute respiratory syndrome (SARS), respiratory complications, asthma, chronic obstructive pulmonary disease (COPD), bronchitis, emphysema, cystic fibrosis, cough, pertussis, pneumonia, pleurisy, bronchiolitis, cold, sinusitis, rhinitis, tracheitis, pharyngitis, laryngitis, acute laryngotracheobronchitis, epiglottitis, bronchiectasis, difficulty breathing, dyspnoea, breathlessness, shortness of breath, fever, fatigue, muscle aches, muscle pain, nasal congestion, runny nose, sore throat, gastrointestinal symptoms, nausea, diarrhoea, renal insufficiency, loss of appetite, general feeling of malaise.

Preferably, said (a) usnic acid of natural origin is a combination or association (C/A) between a dextrorotatory natural usnic acid D(+) and a levorotatory natural usnic acid L(-); preferably the dextrorotatory form D(+) is comprised from 0.1% to 99.9% by weight, with respect to the total weight of the combination or association (C/A), and/or levorotatory form L(-) is comprised from 99.9% to 0.1% by weight, with respect to the total weight of the combination or association.

Preferably, said (a) usnic acid of natural origin is in racemic form 50% (+) and 50% (-), or substantially or at 100% in dextrorotatory form D(+).

Preferably, said (b) a salt of the usnic acid of natural origin is an alkali metal or alkaline-earth metal salt; preferably said usnic acid salt of natural origin is an usnic acid sodium salt.

Preferably, said (b) natural usnic acid salt is in racemic form, or dextrorotatory form D(+); preferably said salt can be present in dextrorotatory form D(+) in an amount by weight comprised from 0.1% to 99.9%, with respect to the total weight of the combination or association (C/A), and/or in levorotatory form L(-) in an amount comprised from 99.9% to 0.1% by weight, with respect to the total weight of the combination or association (C/A).

Preferably, said (a) an usnic acid of natural origin and said (b) a salt thereof are present in a [(a):(b)] by weight ratio comprised from 1:10 to 10:1, preferably from 1:5 to 5:1, even more preferably from 1:3 to 3:1 or 1:1.

Preferably, the usnic acid in the mixture M consists in the compound: (+)-usnic acid, chemical name 2,6-diacetyl-7,9-dihydroxy-8,9b-dimethyldibenzo[b,d]furan-1,3(2H,9bH)-dione; (+)-usnic acid from *Usnea;* CAS N°: 7562-61-0, EC: 231-456-0; preferably the usnic acid sodium salt consists of the compound: monosodium salt of 2,6-diacetyl-7,9-dihydroxy-8,9b-dimethyldibenzo-1,3(2H,9bH)-dione; CAS N°: 34769-44-3, EC: 252-204-6; preferably the purity of said (a) an usnic acid and/or of said (b) an usnic acid salt is comprised from 95% to 99.9%, preferably from 96% to 99.5%, even more preferably from 97% to 98% or 98% (% weight/weight).

Preferably, said mixture M may be in the solid or semi-solid state, in dispersed or suspended form, in the form of a gel, or in the liquid state; preferably, said mixture M may be in the form of flakes, granules, powders, pellets, or it can be an aqueous or hydroalcoholic solution or, in organic solvents.

Preferably, said (a) an usnic acid of natural origin and/or said (b) a salt thereof are in powdered solid form with an average particle size comprised from 1 micron to 100 microns, preferably from 5 microns to 50 microns, even more preferably from 10 microns to 20 microns.

Preferably, said (a) an usnic acid and/or said (b) a salt thereof, is/are extracted from lichens, preferably selected from the group comprising or, alternatively, consisting of *Usnea, Cladonia, Hypotrachyna, Lecanora, Ramalina, Evernia, Parmelia, Alectoria* and combinations thereof, more preferably from Usnea, even more preferably from *Usnea longissima* Ach., and where said at least one cyclodextrin (ii) comprises or, alternatively, consists of at least one beta-cyclodextrin, preferably said at least one beta-cyclodextrin is (2-hydroxypropyl)-β-cyclodextrin.

Preferably, said (a) an usnic acid and/or said (b) a salt thereof, and said (ii) at least one cyclodextrin, preferably at least one beta-cyclodextrin, are in a [(a)and/or(b):(ii)] by weight ratio substantially of 1:1.

Preferably, said inclusion compound (ci) comprises solid particles of said (a) an usnic acid, preferably D(+)-usnic acid as substantially pure enantiomer, or a salt thereof, wherein said solid particles have an average particle size distribution comprised from 0.01 µm to 50 µm, preferably comprised from 0.1 µm to 30 µm, more preferably comprised from 0.15 µm to 20 µm, even more preferably comprised from 0.2 µm to 15 µm. Object of the invention is a composition comprising a mixture M as above described and at least one acceptable pharmaceutical or food grade additive and/or excipient.

Said composition is a pharmaceutical composition, a medical device composition according to Regulation (EU) 2017/745, a composition for a Food for Special Medical Purposes (FSMP), a composition for Novel Food according to Regulation (EU) 2015/2283, or a composition for foodstuffs.

Preferably, the composition is for internal, external, parenteral, topical, dermatological use; or for use on tissues, dermis or mucosa; or for oral, nasal, ocular use; or for use in the auditory canal, throat, trachea, oesophagus, eye, nose or ear; or for use on the oral mucosa, nasal mucosa, ocular mucosa or of the auditory canal; or for use in the stomach, intestine, gastro-intestinal use; or for vaginal or rectal use.

Preferably, the composition is for use as above described, preferably, said composition is:
- in solid form selected from: tablets, chewable tablets, mouth dissolvable tablets, granules, powder, flakes, soluble or water-soluble powder or granules, mouth dissolvable powders or granules, capsules; or
- in liquid form selected from: solutions, suspensions, dispersions, emulsions, liquid which can be dispensed in form of spray, oral spray or nasal spray, aerosol, oral aerosol or nasal aerosol, syrups; or
- in semiliquid or semisolid form selected from: soft-gel, gel, ointment, cream, cream for topical, oral or nasal use.

Preferably, said at least one acceptable pharmaceutical or food grade additive and/or excipient is selected from the group comprising or, alternatively, consisting of: collagen, chondroitin, hyaluronic acid or a salt thereof, preferably sodium hyaluronate, vitamins, amino acids, antioxidants, silica, cellulose, carboxy cellulose, carboxymethyl cellulose (CMC), carboxyethyl cellulose, carboxypropyl cellulose, hydroxypropyl cellulose, hydroxypropylmethyl cellulose (HPMC) poloxamers, fibres, inulin, maltodextrin, fats, waxes, oils, animal oils, vegetable oils, vaseline, carbonate, bicarbonate, ammonium salts, sodium chloride, clays, kaolin.

### EXPERIMENTAL PART (I)

### 1. Study design

The object of the present *in vitro* study is to evaluate the antiviral effects of a complex comprising usnic acid and β-cyclodextrins (inclusion complex according to the present invention) against the SARS-CoV-2 coronavirus.

To this end, the inhibitory effect of said complex according to the present invention against *a Pseudovirus* incubated with a Vero E6 CRL-1586 (ATCC) African green monkey kidney cell line, was tested. At an international level, said Vero E6 cell line is chosen because it is rich in the ACE2 receptor through which the coronaviruses, including *Pseudovirus,* enter the cells. It is known that coronaviruses and the *Pseudovirus* bind to the ACE2 receptor through the surface glycoprotein of the virus referred to as Spike.

The complex according to the present invention which was tested is a usnic acid complex with beta-cyclodextrins (by weight ratio 1:1 and 1:2).

This Vero E6 cell line was primarily used to test the possible cytotoxicity of the complex under analysis.

### 2. cytotoxicity assay of the usnic acid complex and β-cyclodextrin on the Vero E6 cell line.

### 2.1. Culture of a Vero E6 cell line.

A Vero E6 cell line was cultured in Dulbecco's Modification of Eagle's Medium (D-MEM) (Corning) with 1% of L-glutamine, 10% of foetal bovine serum (FBS) and 1% of penicillin and streptomycin (% weight/weight or % weight/volume) and incubated at 37°C in an incubator with a 5% CO₂ atmosphere.

Cell propagation is carried out in 75 cm² flasks containing 10 ml of cell culture medium in order to obtain a number of cells sufficient to inoculate 96-well plates in which the cells were subjected to the cytotoxicity assay.

### 2.2. Solubility of the usnic acid and β-cyclodextrins complex.

Before carrying out cytotoxicity assay, solubility tests of the usnic acid and β-cyclodextrins complex were carried out, both in sterile distilled water and in Dulbecco's Modified Eagle Medium (D-MEM, Corning).

The solubility of the complex is equal to 1% weight/weight in demineralised water at T 20°C. An energetic mixing, for example a mechanical mixing, is required in order to obtain a good dispersion of the complex. The solution thus obtained can form a slow precipitate. For a better solubilisation, it is recommended to submerge the 1% solution in water at 50°C in order to allow a better solubility of the complex, also with the aid of a mixing, for example of the mechanical type.

In order to verify the solubility of the usnic acid and β-cyclodextrins complex in sterile distilled water and in D-MEM, the following concentrations were tested for subsequent use in the cytotoxicity assay:
- 1% usnic acid and β-cyclodextrins complex corresponding to the concentration of 10 mg/ml;
- 0.5% usnic acid and β-cyclodextrins complex corresponding to the concentration of 5 mg/ml;
- 0.1% usnic acid and β-cyclodextrins complex corresponding to the concentration of 1 mg/ml;
- 0.05% usnic acid and β-cyclodextrins complex corresponding to the concentration of 500 µg/ml;
- 0.01% usnic acid and β-cyclodextrins complex corresponding to the concentration of 100 µg/ml;
- 0.005% usnic acid and β-cyclodextrins complex corresponding to the concentration of 50 µg/ml;
- 0.001% usnic acid and β-cyclodextrins complex corresponding to the concentration of 10 µg/ml.

The solubility tests showed that no solubility differences were observed both in the distilled water and in the culture medium used. As a result, the D-MEM cell culture medium was used for the dissolution of the complex to be used for the cytotoxicity assay. This procedure proved to be optimal because the cells maintain their viability in the D-MEM cell culture medium.

The dissolution of the usnic acid and β-cyclodextrins complex dissolved D-MEM, at concentrations of 1%, 0.5% and 0.1%, showed the formation of a slow precipitate proportional to the concentration of the complex tested. Therefore, at these concentrations only the supernatant was collected and incubated with the Vero E6 cell line. Lower concentrations and that is 0.05%, 0.01%, 0.005% and 0.001% did not show the presence of precipitates and therefore there was no need to collect the supernatant.

### 2.3. Cytotoxicity assay (MTT assay)

In order to determine the non-cytotoxic concentration of the usnic acid and β-cyclodextrins complex, the cytotoxicity test of this complex was carried out at different concentrations and at two incubation times on the Vero E6 cell line.

Thiazolyl blue tetrazolium bromide or MTT (Sigma Aldrich) was used to check the possible cytotoxic activity of the usnic acid and β-cyclodextrins complex at different concentrations. The MTT assay is a colorimetric assay based on the reduction - by the NAD(P)H-dependent oxidoreductase enzyme in viable cells - of the yellow tetrazolium bromide compound, into formazan, which precipitates in the viable cells in the form of insoluble purple crystals. The higher the purple colour observed, the higher the number of viable cells present.

In detail, 96-well multiwells in which the cells - previously removed from the 75 cm² flasks (Corning) - were seeded at a final concentration of 1x10⁴ cells/well, were used for the cytotoxicity experiment. Subsequently, the multiwells containing the cells were incubated for 24 hours at 37°C in a humidified incubator with a 5% CO₂ atmosphere. After the 24 hours of incubation, the culture medium was removed and 100 µl of the usnic acid and β-cyclodextrins complex were added at different concentrations such as 1%; 0.5%; 0.1%; 0.05%; 0.01%, 0.005% and 0.001%, , previously dissolved in D-MEM (paragraph 2.2.). The multiwells were therefore incubated for 8 hours and 24 hours at 37°C in a humidified incubator with a 5% CO₂ atmosphere After 8 hours and 24 hours of incubation, the culture medium with the different concentrations of the usnic acid and β-cyclodextrin complex was removed from the multiwells and a washing was carried out with 100 µl/well of phosphate buffered saline (PBS).

At this point, the MTT assay was conducted for the cytotoxicity test.

In the absence of light sources, 100 µl/well of a 0.5 mg/ml solution of MTT dissolved in PBS were added and the multiwells were incubated at 37°C in a humidified incubator with a 5% CO₂ atmosphere in the dark for 3 hours.

After 3 hours, the MTT solution was removed from the multiwells, and a PBS wash was carried out, after which 100 µl/well of DMSO were added. The multiwells were incubated for 15 minutes at room temperature in the dark. Then, the spectrophotometer reading was carried out at the wavelength of 570 nm.

### 2.4. Results of the cytotoxicity test.

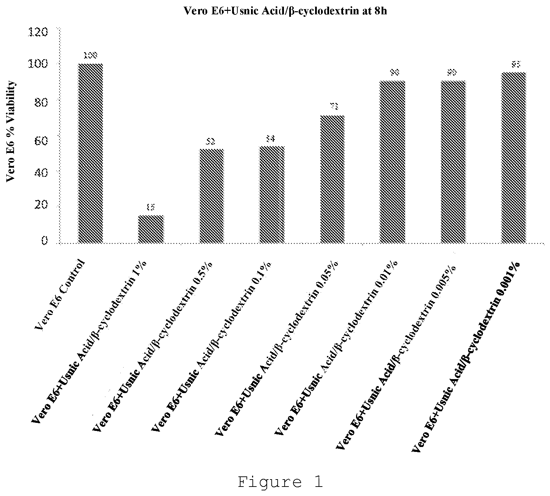

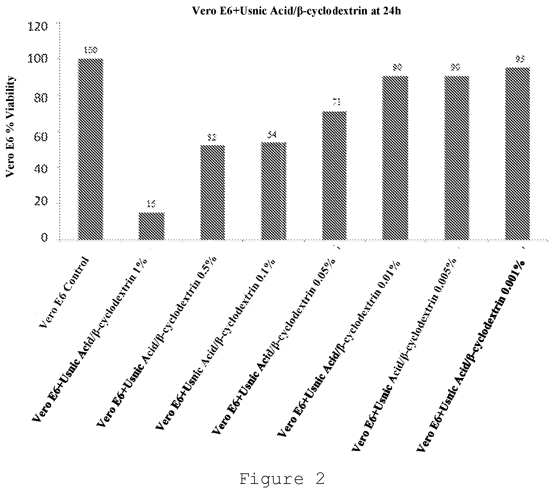

The results obtained from the cytotoxicity test are reported in Figure 1 and in Figure 2, respectively for the 8 hours and 24 hours of incubation of the usnic acid and β-cyclodextrins complex with the Vero E6 cells.

In Figures 1 and 2 the cell viability is expressed as a percentage. A cell viability in the presence of the complex of lower than about 70% classifies the complex as cytotoxic at that concentration.

As observable from Figures 1 and 2, the usnic acid and β-cyclodextrins complex, at the concentrations of 1%, 0.5% and 0.1%, is cytotoxic for Vero E6 cells both after 8 hours and after 24 hours of incubation.

As regards the cytotoxicity of the usnic acid and β-cyclodextrin complex at the concentration of 0.05%, it is non-cytotoxic (71% of viability) after 8 hours of incubation (Figure 1); after 24 hours of incubation, cell viability drops slightly below 70% (Figure 2).

As regards the other concentrations of the usnic acid and β-cyclodextrins complex assayed, that is from 0.01 to 0.001%, these concentrations are not cytotoxic for the Vero E6 cell line both after 8 hours and after 24 hours of incubation (Figures 1 and 2).

Therefore, the antiviral activity of the complex will be tested at the concentration of 0.01%.

### 3. Antiviral activity of the usnic acid and β-cyclodextrins complex against Pseudovirus containing the SARS-CoV-2 spike protein tested on the Vero E6 cell line.

### 3.1. Vero E6 cell line culture

The possible antiviral activity of the usnic acid and β-cyclodextrins complex against *Pseudovirus* containing the SARS-CoV-2 spike protein, was tested at the concentration of 0.01%.

As with the cytotoxicity assay, the African green monkey kidney cell line Vero E6 CRL-1586 (ATCC) was also used for antiviral activity. This cell line was cultured in Dulbecco's Modification of Eagle's Medium (D-MEM) (Corning) with 1% of L-glutamine, 10% of foetal bovine serum (FBS) and 1% of penicillin and streptomycin (% weight/weight or % weight/volume) and incubated at 37°C in an incubator with a 5% CO₂ atmosphere.

Cell propagation is carried out in 75 cm² flasks containing 10 ml of cell culture medium in order to obtain a number of cells sufficient to inoculate 96-well plates in which the cells will be subjected to the test for antiviral activity of the usnic acid and β-cyclodextrins complex.

### 3.2. Dissolution of the usnic acid and β-cyclodextrins complex.

The usnic acid and β-cyclodextrin complex was dissolved in D-MEM (cell culture medium) at 0.01%, corresponding to a concentration equal to 100 µg/ml.

### 3.3. Pseudovirus.

The *Pseudovirus* stock containing the SARS-CoV-2 spike protein (Creative Biogene, USA), used for the present experiment, contains 10⁷ transduction units (TUs)/ml, where TUs are the unit of measurement used to define the amount *of Pseudoviruses.* It should be observed that the *Pseudovirus* used in this experiment also contains the reporter gene for the firefly luciferase enzyme which will allow to quantify the luminescence emitted by the viral particles that infected the cell monolayer.

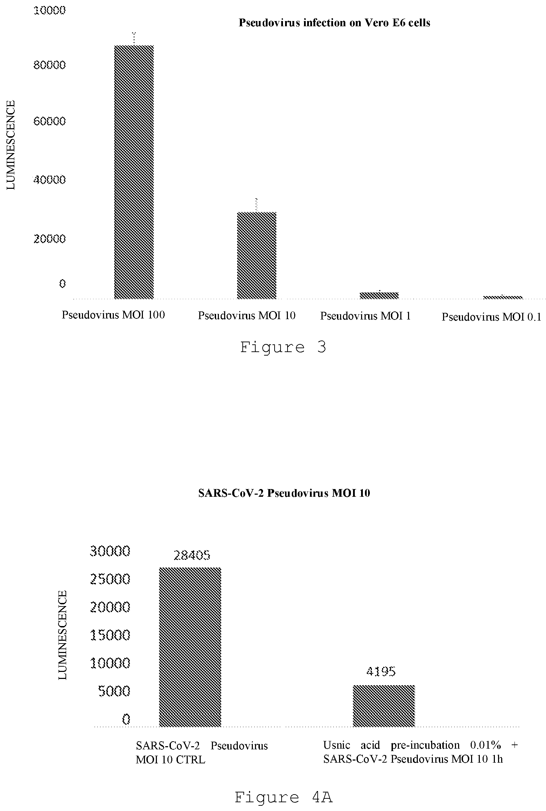

In the experiment carried out, we used the concentration of 10⁵ TU, corresponding to a multiplicity of infection (MOI) of 10. Multiplicity of infection indicates how many viral particles are contacted with a known number of cells per well (1x10⁴). Therefore, in the present experiment 10 *Pseudovirus* particles per cell (MOI 10) were contacted after testing various MOls (Figure 3). As observable from Figure 3, the optimal concentration is the one obtained with MOI 10 given that lower concentrations do not fall within the linear area of the standard curve same case applying to the excess luminescence observed at MOI 100.

### 3.4. Antiviral activity assay.

Vero E6s are the optimal cell line for the study of viral infection given that they have the ACE2 receptor for the attachment of the SARS-CoV-2 spike protein and the subsequent entry of the virus into the cell. In detail, 96-well multiwells in which the cells - previously removed from the 75 cm² flasks (Corning) - were seeded at a final concentration of 1x10⁴ cells/well D-MEM, were used for the antiviral activity experiment. Subsequently, the multiwells containing the cells were incubated for 24 hours at 37°C in a humidified incubator with a 5% CO₂ atmosphere.

The following day, cell count was carried out, verifying the non-replication thereof, and the *Pseudovirus* containing the SARS-CoV-2 spike protein was pre-incubated for 1 hour at 37°C, in a humidified incubator with a 5% CO₂, at MOI 10 with the usnic acid and β-cyclodextrins complex at a concentration of 0.01%. Pre-incubation was carried out in 1.5 ml test tubes.

When the contact time between the *Pseudovirus* and the usnic acid and β-cyclodextrins complex ended, the whole suspension was collected from the test tube and added to the cell monolayer of Vero E6 cells. The monolayer was incubated for 8 hours at 37°C in a humidified incubator with a 5% CO₂ atmosphere.

After 8 hours of incubation, the culture medium containing the usnic acid and β-cyclodextrins complex with *Pseudovirus* was removed from the multiwells and a washing with 100 µl/well of phosphate buffered saline (PBS), sufficient to remove the virus that had not penetrated into the cells, was carried out.

Subsequently, a new D-MEM medium was added to the monolayer and incubated for 48 hours at 37°C in a humidified incubator with a 5% CO₂ atmosphere.

After this period of time had expired, the cell monolayer was lysed using the *Luciferase Cell Culture Lysis* Reagent (Promega, Italy). The luciferase test (Promega, Italy) was then carried out. The luciferase test is based on the oxidation of luciferin to oxyluciferin, using the firefly luciferase enzyme cloned in the *Pseudovirus.* The products of this reaction emit a light signal, due to an electronic transition, which is detected by a luminometer (Biontek).

As a result, the intensity of light emitted will be directly proportional to the number of *Pseudovirus* particles that infected the cell monolayer, and, therefore, inversely proportional to the affinity of the complex of the present invention toward the *Pseudovirus* (the *Pseudovirus* Spike).

The amount of luminescence emitted by the samples in which the *Pseudovirus* was pre-incubated with the usnic acid and β-cyclodextrin complex was compared with the amount of luminescence obtained with *the Pseudovirus* incubated at 37°C in the absence of the usnic acid and β-cyclodextrin complex (control).

### 3.5. Results of the antiviral activity assay.

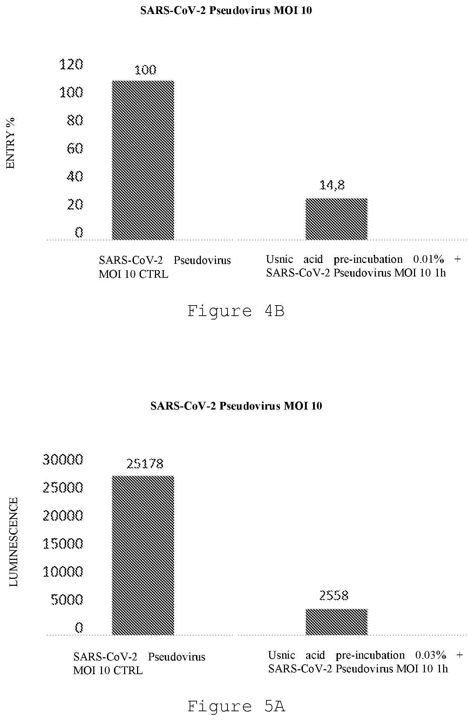

The results obtained from the test for antiviral activity of the usnic acid and β-cyclodextrins complex, at the concentration of 0.01%, pre-incubated for 1 hour with the *Pseudovirus* at MOI 10 and subsequently contacted with Vero E6 cells for 8 hours are reported in Figure 4.

The results of the samples in which the *Pseudovirus* was pre-incubated with the usnic acid and β-cyclodextrins complex were compared with the results obtained from *Pseudovirus* not pre-incubated with the virucidal complex (control). The data are reported both in the amount of luminescence emitted (Figure 4A) and in the rate of entry of the *Pseudovirus* into the cell monolayer (Figure 4B).

As observable from Figure 4, when the *Pseudovirus* at MOI 10 is contacted with the usnic acid and β-cyclodextrins complex at 0.01%, there is a decrease in luminescence with respect to the highly accentuated control (4195 vs 28405, respectively) (Figure 4A). This indicates a decrease in *Pseudovirus* entry and an 85.2% inhibition rate of entry of the virus into the cells with respect to the control (Figure 4B).

In conclusion, the illustrated experiments showed a virucidal activity of the usnic acid and β-cyclodextrins complex at a non-cytotoxic concentration equal to 0.01% against the Pseudovirus containing the SARS-CoV-2 spike protein.

### EXPERIMENTAL PART (II)

### 1. Purpose of the experiment

The cytotoxicity assay of the usnic acid and β-cyclodextrins complex (1:1) on the Vero E6 cell line and the antiviral activity assay of said complex against the Pseudovirus containing the SARS-CoV-2 spike protein on the Vero E6 cell line, as reported in the Experimental Part (I), were repeated using the usnic acid and β-cyclodextrins complex (1:1) at a concentration - in D-MEM cell culture medium - of 0.03% (corresponding to a concentration of 300 µg/ml) and at a concentration of 0.05% (corresponding to a concentration of 500 µg/ml).

### 2. Cytotoxicity assay (MTT assay)

Cytotoxicity assays for the usnic acid and β-cyclodextrins complexes (1:1) at a concentration of 0.03% and 0.05% (inclusion complexes according to the present invention) were conducted exactly under the conditions reported in paragraphs 2.1.-2.3. of the Experimental part (I).

The cell viability rate of the Vero E6 cell line after 8 hours of incubation with the usnic acid and β-cyclodextrins complex (1:1) at the concentration 0.03% was found to be 83%.

The cell viability rate of the Vero E6 cell line after 8 hours of incubation with the usnic acid and β-cyclodextrins complex (1:1) at the concentration 0.05% was found to be 68% in a first test and 69% in a second test (values stable at the 70% viability threshold established as the minimum value for defining the non-cytotoxic concentration).

In the light of the above, it was established that both the concentration of 0.03% and the concentration of 0.05% of the usnic acid and β-cyclodextrins complex (inclusion complexes according to the present invention) are not cytotoxic for the Vero E6 cell line after 8 hours of incubation.

### 3. Antiviral activity assay.

The antiviral activity assays for the usnic acid and β-cyclodextrins complexes (1:1) at a concentration of 0.03% and 0.05% (inclusion complexes according to the present invention) were conducted exactly under the conditions reported in paragraphs 3.1.-3.4 of the Experimental part (I).

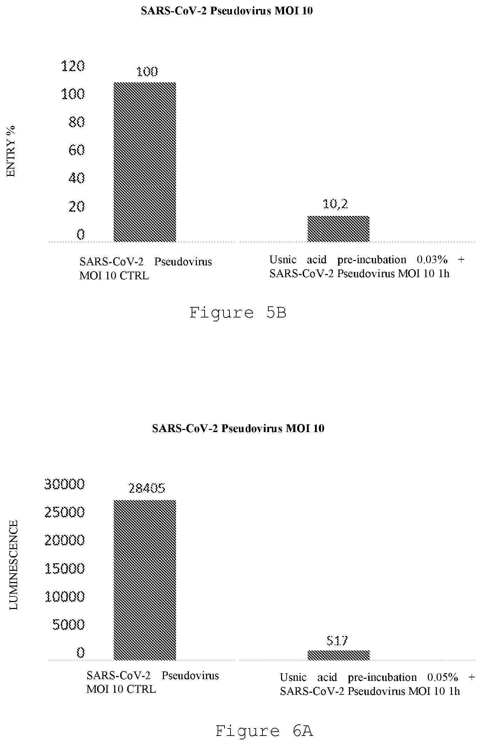

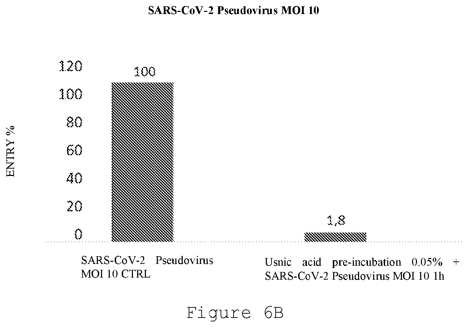

The results obtained from the test for antiviral activity of the usnic acid and β-cyclodextrins complex, at the concentration of 0.03% 0.05%, pre-incubated for 1 hour with the Pseudovirus at MOI 10 and subsequently contacted with Vero E6 cells for 8 hours are reported in Figures 5 and 6.

The results of the samples in which the Pseudovirus was pre-incubated with the usnic acid and β-cyclodextrins complex were compared with the results obtained from Pseudovirus not pre-incubated with the virucidal complex (control). The data are reported both in the amount of luminescence emitted (Figures 5A and 6A) and in the rate of entry of the Pseudovirus into the cell monolayer (Figures 5B and 6B).

As observable from Figure 5, when the Pseudovirus at MOI 10 is contacted with the usnic acid and β-cyclodextrins complex at 0.03%, there is a decrease in luminescence with respect to the highly accentuated control (2558 vs 25178, respectively) (Figure 5A). This indicates a decrease in *Pseudovirus* entry and an 89.8% inhibition rate of entry of the virus into the cells with respect to the control (Figure 5B).

As observable from Figure 6, when the Pseudovirus at MOI 10 is contacted with the usnic acid and β-cyclodextrins complex at 0.05%, there is a decrease in luminescence with respect to the highly accentuated control (398 vs 1515, respectively) (Figure 6A). This indicates a decrease in *Pseudovirus* entry and an 73.7% inhibition rate of entry of the virus into the cells with respect to the control (Figure 6B).

In conclusion, the illustrated experiments showed a virucidal activity of the usnic acid and β-cyclodextrins complex at a non-cytotoxic concentration equal to 0.03% and 0.05% against the *Pseudovirus* containing the SARS-CoV-2 spike protein.

The compositions reported below in the table were tested by the Applicant to evaluate, among other things, cell viability, in particular of nasal cells, protection of nasal epithelium and mitochondrial balance, as well as the determination of the ability of the compositions to prevent the entry of the SARS-CoV-2 virus into the nasal epithelium which is the first barrier involved in Covid-19 infection.

| | |
|---|---|
| P21H006 | 150 mcg/ml usnic acid + 150 mcg/ml of beta-cyclodextrin, without preservative |
| P21H007 | 150 mcg/ml usnic acid + 150 mcg/ml of beta-cyclodextrin, with preservative |
| P21H008 | 75 mcg/ml usnic acid + 150 mcg/ml of beta-cyclodextrin, with preservative |
| P21H008 | 75 mcg/ml usnic acid + 150 mcg/ml of beta-cyclodextrin, with preservative + coenzyme Q10 |
| P21H008 | 75 mcg/ml usnic acid + 150 mcg/ml of beta-cyclodextrin with preservative + Quercetin |
| P21H009 | 150 mcg/ml of beta-cyclodextrin, with preservative |
| P21H0013 | 75 mcg/ml usnic acid + 150 mcg/ml of beta-cyclodextrin, with preservative + Glutathione |
| P21H0014 | 75 mcg/ml usnic acid + 150 mcg/ml of beta-cyclodextrin, with preservative + Tocopherols, or mixture of Tocopherols and Tocotrienols |

All the formulations reported above in the table showed good results.

## Claims

1. A mixture M for use in a method for the treatment of a viral infection triggered or caused by at least one coronavirus,
wherein said mixture M comprises or, alternatively, consists of:
an inclusion compound (ci), said inclusion compound (ci) comprising or, alternatively, consisting of:
- (i) (a) an usnic acid and/or (b) a salt thereof, and
- (ii) at least one cyclodextrin.

2. The mixture M for use according to claim 1, wherein said mixture M is for use in a method for the treatment of viral infections of the respiratory system triggered or caused by a SARS-CoV-2 virus; preferably said mixture M is capable of inhibiting the spike protein of said SARS-CoV-2 virus binding with at least one SARS-CoV-2 protease and/or one SARS-CoV-2 spike protein, preferably with the SARS-CoV-2 RBD spike protein.

3. The mixture M for use according to claim 1 or 2,
wherein said inclusion compound (ci) comprises or, alternatively, consists of:
- (i) said (a) usnic acid and/or a usnic acid sodium salt, and
- (ii) at least one beta-cyclodextrin.

4. The mixture M for use according to any one of the preceding claims, wherein in said inclusion compound (ci) said (ii) at least one cyclodextrin, preferably at least one beta-cyclodextrin, and said (i) usnic acid and/or salt thereof are in a [(ii):(i)] by weight ratio comprised from 3:1 to 1:3, preferably comprised from 2:1 to 1:2, more preferably comprised from 1.5:1 to 1:1.5, even more preferably being 2:1, or 1:1.

5. The mixture M for use according to any one of the preceding claims, wherein said (a) usnic acid is of natural origin and is a combination or association (C/A) between a dextrorotatory natural usnic acid D(+) and a levorotatory natural usnic acid L(-); preferably the dextrorotatory form D(+) is comprised from 0.1% to 99.9% by weight, with respect to the total weight of the combination or association (C/A), and/or the levorotatory form L(-) is comprised from 99.9% to 0.1% by weight, with respect to the total weight of the combination or association, preferably wherein said (a) usnic acid of natural origin is in racemic form 50% (+) and 50% (-), or substantially equal to 100% in dextrorotatory form D(+).

6. The mixture M for use according to any one of the preceding claims, wherein said (a) usnic acid of natural origin is an alkali metal or alkaline-earth metal salt; preferably said usnic acid salt of natural origin is a usnic acid sodium salt D(+).

7. The mixture M for use according to any one of the preceding claims, wherein said (a) usnic acid essentially consists of the compound: (+)-usnic acid, chemical name 2,6-diacetyl-7,9-dihydroxy-8,9b-dimethyldibenzo[b,d]furan-1,3(2H,9bH)-dione; (+)-usnic acid from *Usnea;* CAS N°: 7562-61-0, EC: 231-456-0; preferably the usnic acid sodium salt essentially consists of the compound: monosodium salt of 2,6-diacetyl-7,9-dihydroxy-8,9b-dimethyldibenzo-1,3(2H,9bH)-dione; CAS N°: 34769-44-3, EC: 252-204-6; preferably the purity of said (a) an usnic acid and/or of said (b) an usnic acid salt is comprised from 95% to 99.9%, preferably from 96% to 99.5%, even more preferably from 97% to 98% or 99% (% weight/weight).

8. The mixture M for use according to any one of the preceding claims, wherein said (a) usnic acid of natural origin and/or said (b) a salt thereof are in powdered solid form with an average particle size comprised from 1 micron to 100 microns, preferably from 5 microns to 50 microns, even more preferably from 10 microns to 20 microns.

9. The mixture M for use according to any one of the preceding claims, wherein said (a) usnic acid and/or said (b) a salt thereof, and said (ii) at least one cyclodextrin, preferably at least one beta-cyclodextrin, are in a [(a)and/or(b):(ii)] by weight ratio substantially of 1:2, or 1:1.

10. The mixture M for use according to any one of the preceding claims, wherein said inclusion compound (ci) comprises solid particles of said (a) usnic acid, preferably D(+)-usnic acid as substantially pure enantiomer, or salt thereof, wherein said solid particles have an average particle size distribution comprised from 0.01 µm to 50 µm, preferably comprised from 0.1 µm to 30 µm, more preferably comprised from 0.15 µm to 20 µm, even more preferably comprised from 0.2 µm to 15 µm.

11. A pharmaceutical composition, a medical device composition according to Regulation (EU) 2017/745, a composition for a Food for Special Medical Purposes (FSMP), a composition for Novel Food according to Regulation (EU) 2015/2283, or a composition for foodstuffs for use in a method for the treatment of a viral infection triggered or caused by at least one coronavirus, wherein said composition for use comprises a mixture M according to any one of claims 1 to 10 and at least one acceptable pharmaceutical or food grade additive and/or excipient, wherein said composition is for internal, external, parenteral, topical, dermatological use; or for use on tissues, dermis or mucosa; or for oral, nasal, ocular use; or for use in the ear canal, throat, trachea, oesophagus, eye, nose or ear; or for use on the oral mucosa, nasal mucosa, nasal epithelium, ocular mucosa or of the ear canal; or for use in the stomach, bowel gastro-intestinal use; or for vaginal or rectal use.

12. The composition for use according to claim 11, wherein said composition is:
- in solid form selected from: tablets, chewable tablets, mouth dissolvable tablets, granules, powder, flakes, soluble or water-soluble powder or granules, mouth dissolvable powders or granules, capsules; or
- in liquid form selected from: solutions, suspensions, dispersions, emulsions, liquid which can be dispensed in the form of spray, throat spray, oral spray or nasal spray, aerosol, oral aerosol or nasal aerosol, syrups; or
- in semiliquid or semisolid form selected from: soft- gel, gel, nasal gel, oral gel, ointment, cream, cream or gels for topical, oral or nasal use.

13. The composition for use according to claim 11 or 12, wherein said composition is formulated by using at least one acceptable pharmaceutical or food grade additive and/or excipient which is selected from: collagen, chondroitin, hyaluronic acid or a salt thereof, such as sodium hyaluronate, vitamins, amino acids, antioxidants, silica, cellulose, carboxy cellulose, carboxymethyl cellulose (CMC), carboxyethyl cellulose, carboxypropyl cellulose, hydroxypropyl cellulose (HPC), hydroxypropyl methylcellulose (HPMC), hydroxymethyl cellulose, hydroxyethyl cellulose, hydroxypropyl cellulose, poloxamers, fibres, inulin, maltodextrin, fats, waxes, oils, animal oils, vegetable oils, vaseline, carbonate, bicarbonate, ammonium salts, sodium chloride, clays, kaolin, antioxidants such as for example glutathione, quercetin, coenzyme Q10 or vitamins, in particular vitamins of group C or E, such as for example tocopherols and tocotrienols, tocotrienols of plant origin such as for example tocotrienols from rice, preservatives such as for example sodium and potassium benzoate, or sodium or potassium sorbate, mineral salts, sodium chloride, and pH buffers such as for example citric acid and a soluble salt thereof, for example citric acid monohydrate.

14. The composition for use according to any one of claims 11-13, wherein said composition is an aqueous liquid solution which is administered using an atomiser or sprayer or spray can; preferably said composition is formulated using food or pharmaceutical grade additives and/or excipients which allow, once the liquid composition, for example at a room temperature of about 20°C or 5°C, is applied through the nasal route to a subject with a body temperature for example comprised from 36°C to 40°C, to bring the liquid aqueous composition to room temperature to take a consistency of a gel.

15. The composition for use according to claim 14, wherein said composition is used in an aqueous composition to be atomised, combined or associated with hydroxypropyl methylcellulose (HPMC) and tocotrienols, preferably tocotrienols from rice; preferably, tocotrienols from rice *(Oryza sativa* L.) is in the form of a water-dispersible powder for example a tocotrienol content comprised from 5% to 75%, preferably from 15% to 60%, for example 25%, or 30%, or 40%, or 50% by weight.

16. The composition for use according to claim 15, wherein said water-dispersible powder contains, together with the tocotrienols, even preferably maltodextrin (9050-36-6), starch and silicon dioxide SiO2 (7631-86-9); preferably, the water-disposable powder contains a rice bran oil extract from 30% to 60%, modified starch from 25% to 45%, maltodextrin from 20% to 40% and SiO2 from 0.2 to 1.5%; even more preferably, a water-dispersible powder containing a mixture of tocotrienols and tocopherols in a total amount by weight comprised from 15% to 60%, for example 25%, or 30%, or 40%, or 50% is used; even more preferably in the case of a total content in the mixture of 25% by weight, there is for example an amount of about 10% by weight of tocotrienols and an amount of about 15% by weight of tocopherols (amount measured by means of HPLC), while in the case of a total content in the mixture of 30% by weight there is about 15% by weight of tocotrienols and about 15% by weight of tocopherols, the remaining part at 100% is starch, maltodextrins, SiO2 and other.

17. The composition for use according to any one of claims 11 to 16, wherein the usnic acid D(+) has a melting point (DSC) comprised from 190°C to 210°C, preferably from 192°C to 198°C; a molecular rotation comprised from 480° to 540°, preferably from 490° to 520°; a PSD value: D50 comprised from 1 micron to 15 microns, preferably from 2 microns to 10 microns, for example 5 microns; even more preferably said usnic acid has a low content of pathogens such as *Staffilococco Aureus* MIC less than or equal to 500 ppm and *Escherichia Coli* MIC less than or equal to 1000 ppm.

18. The composition for use according to any one of claims 11 to 17, wherein said composition comprises:
| Ingredients | Compositions (from x% to y%) | | | |
|---|---|---|---|---|
| Water | 94-99 | 95-98 | 96-97.8 | Aqueous liquid solutions at 20°C |
| CMC, or HPC, or HPMC | 0.25-2.5 | 0.5-1.5 | 0.65-0.95 | |
| Usnic acid, or a sodium salt | 0.0001-0.5 | 0.0005-0.05 | 0.001-0.0075 | |
| Beta-cyclodextrin | 0.005-0.5 | 0.0075-0.25 | 0.01-0.015 | |
| NaCl | 0.1-2 | 0.3-1.5 | 0.5-0.9 | |
| Sodium Benzoate | 0.001-1 | 0.01-0.75 | 0.05-0.1 | |
| Potassium Sorbate | 0.001-1 | 0.01-0.75 | 0.05-0.1 | |
| Tocotrienols, or mixture of tocotrienols and tocopherols | 0.001-1 | 0.01-0.75 | 0.05-0.1 | |
| Citric acid | 0.001-0.1 | 0.01-0.005 | 0.015-0.02 | |
| Total weight (w/w) | 100 | | | |
| Density 1g/cc | | | | |

19. The composition for use according to any one of claims 11 to 18, wherein said composition in the form of aqueous solution comprises or, alternatively, consists of:
- water, preferably demineralised water, in an amount by weight comprised from 90% to 99.5%, preferably from 94% to 99%;
- an inclusion compound (ci), wherein said (a) usnic acid is preferably in the form D(+), in an amount by weight comprised from 0.0001% to 0.5%, preferably from 0.01 to 0.25%;
- a polymer selected from the group comprising or, alternatively, consisting of: cellulose, carboxy cellulose, carboxymethyl cellulose (CMC), carboxyethyl cellulose, carboxypropyl cellulose, hydroxypropyl cellulose (HPC), hydroxypropyl methylcellulose (HPMC), hydroxymethyl cellulose, hydroxyethyl cellulose, hydroxypropyl cellulose, poloxamers, preferably hydroxypropyl cellulose (HPMC), in an amount by weight comprised from 0.25% to 2.5%, preferably from 0.5% to 1.5%;
- a compound with antioxidant activity selected from the group comprising or, alternatively, consisting of: glutathione, quercetin, coenzyme Q10, vitamins, in particular the vitamins of group C or E, such as for example tocopherols and tocotrienols, preferably tocotrienols of plant origin such as for example tocotrienols from rice;
- preferably mineral salts, preservative agents and pH buffers or stabilisers commonly used in the industry.

20. The composition for use according to claim 19, wherein said composition in the form of an aqueous solution has a viscosity value at 20°C (mPa•second) of about 240 +/- 40; a pH value of about 5 +/- 0.2; a density value g/cc of about 1.01 +/- 0.1 and a microbiological load (total load, yeasts, moulds, 2 pathogens (*Staphilococco Aureus* and *Pseudomonas))* of the type: Total load <= 100 CFUs, yeasts <= 100 CFUs, Moulds <= 10 CFUs and absence of pathogens.

## Patentansprüche

1. Mischung M zur Verwendung in einem Verfahren zur Behandlung einer Virusinfektion, die durch mindestens ein Coronavirus ausgelöst oder verursacht wird,
wobei die Mischung M umfasst oder alternativ besteht aus:
eine(r) Einschlussverbindung (ci), wobei die Einschlussverbindung (ci) umfasst oder alternativ besteht aus:
- (i) (a) eine(r) Usninsäure und/oder (b) ein(em) Salz davon und
- (ii) mindestens ein(em) Cyclodextrin.

2. Mischung M zur Verwendung nach Anspruch 1, wobei die Mischung M zur Verwendung in einem Verfahren zur Behandlung von Virusinfektionen des Atmungssystems ist, die durch ein SARS-CoV-2-Virus ausgelöst oder verursacht werden; vorzugsweise ist die Mischung M in der Lage, das Spike-Protein der SARS-CoV-2-Virusbindung mit mindestens einer SARS-CoV-2-Protease und/oder einem SARS-CoV-2-Spike-Protein, vorzugsweise mit dem SARS-CoV-2-RBD-Spike-Protein, zu hemmen.

3. Mischung M zur Verwendung nach Anspruch 1 oder 2,
wobei die Einschlussverbindung (ci) umfasst oder alternativ besteht aus:
- (i) die(der) (a) Usninsäure und/oder ein(em) Usninsäure-Natriumsalz, und
- (ii) mindestens ein(em) Beta-Cyclodextrin.

4. Mischung M zur Verwendung nach einem der vorhergehenden Ansprüche, wobei in der Einschlussverbindung (ci) das (ii) mindestens ein Cyclodextrin, vorzugsweise mindestens ein Beta-Cyclodextrin, und die (i) Usninsäure und/oder das Salz davon in einem [(ii):(i)] Gewichtsverhältnis von 3:1 bis 1:3, bevorzugt von 2:1 bis 1:2, bevorzugter von 1,5:1 bis 1:1,5, noch bevorzugter von 2:1 oder 1:1 vorliegen.

5. Mischung M zur Verwendung nach einem der vorhergehenden Ansprüche, wobei die (a) Usninsäure natürlichen Ursprungs ist und eine Kombination oder Assoziation (C/A) zwischen einer rechtsdrehenden natürlichen Usninsäure D(+) und einer linksdrehenden natürlichen Usninsäure L(-) ist; vorzugsweise beträgt die rechtsdrehende Form D(+) von 0,1 bis 99,9 Gew.- %, bezogen auf das Gesamtgewicht der Kombination oder Assoziation (C/A), und/oder beträgt die linksdrehende Form L(-) von 99,9 bis 0,1 Gew.- %, bezogen auf das Gesamtgewicht der Kombination oder Assoziation, wobei vorzugsweise die (a) Usninsäure natürlichen Ursprungs zu 50 % (+) und 50 % (-) in racemischer Form oder im Wesentlichen zu 100 % in rechtsdrehender Form D(+) vorliegt.

6. Mischung M zur Verwendung nach einem der vorhergehenden Ansprüche, wobei die (a) Usninsäure natürlichen Ursprungs ein Alkalimetall- oder Erdalkalimetallsalz ist; vorzugsweise ist das Usninsäure-Salz natürlichen Ursprungs ein Usninsäure-Natriumsalz D(+).

7. Mischung M zur Verwendung nach einem der vorhergehenden Ansprüche, wobei die (a) Usninsäure im Wesentlichen aus der Verbindung besteht: (+)-Usninsäure, chemische Bezeichnung 2,6-Diacetyl-7,9-dihydroxy-8,9b-dimethyldibenzo[b,d] furan-1,3 (2H,9bH)-dion; (+)-Usninsäure aus *Usnea;* CAS-Nr.: 7562-61-0, EG: 231-456-0; vorzugsweise besteht das Usninsäure-Natriumsalz im Wesentlichen aus der Verbindung: Mononatriumsalz des 2,6-Diacetyl-7,9-dihydroxy-8,9b-dimethyldibenzo-1,3(2H,9bH)-dions; CAS-Nr.: 34769-44-3, EG: 252-204-6; vorzugsweise beträgt die Reinheit der (a) einen Usninsäure und/oder des (b) einen Usninsäuresalzes von 95 % bis 99,9 %, vorzugsweise von 96 % bis 99,5 %, noch bevorzugter von 97 % bis 98 % oder 99 % (Gew.-%/Gewicht).

8. Mischung M zur Verwendung nach einem der vorhergehenden Ansprüche, wobei die (a) Usninsäure natürlichen Ursprungs und/oder das (b) Salz davon in pulverisierter fester Form mit einer durchschnittlichen Partikelgröße von 1 Mikrometer bis 100 Mikrometer, bevorzugt von 5 Mikrometer bis 50 Mikrometer, noch bevorzugter von 10 Mikrometer bis 20 Mikrometer vorliegen.

9. Mischung M zur Verwendung nach einem der vorhergehenden Ansprüche, wobei die (a) Usninsäure und/oder das (b) Salz davon und das (ii) mindestens ein Cyclodextrin, vorzugsweise mindestens ein Beta-Cyclodextrin, in einem [(a)und/oder(b):(ii)] Gewichtsverhältnis im Wesentlichen von 1:2 oder 1:1 vorliegen.

10. Mischung M zur Verwendung nach einem der vorhergehenden Ansprüche, wobei die Einschlussverbindung (ci) feste Partikel der (a) Usninsäure, vorzugsweise D(+)-Usninsäure als im Wesentlichen reines Enantiomer, oder ein Salz davon umfasst, wobei die festen Partikel eine durchschnittliche Partikelgrößenverteilung von 0,01 µm bis 50 µm, bevorzugt von 0,1 µm bis 30 µm, bevorzugter von 0,15 µm bis 20 µm, noch bevorzugter von 0,2 µm bis 15 µm aufweisen.

11. Pharmazeutische Zusammensetzung, Zusammensetzung für medizinische Geräte gemäß der Verordnung (EU) 2017/745, Zusammensetzung für ein Lebensmittel für besondere medizinische Zwecke (FSMP), Zusammensetzung für neuartige Lebensmittel gemäß der Verordnung (EU) 2015/2283 oder Zusammensetzung für Lebensmittel zur Verwendung in einem Verfahren zur Behandlung einer Virusinfektion, die durch mindestens ein Coronavirus ausgelöst oder verursacht wird, wobei die Zusammensetzung zur Verwendung eine Mischung M nach einem der Ansprüche 1 bis 10 und mindestens einen akzeptablen Zusatzstoff und/oder Hilfsstoff in pharmazeutischer oder Lebensmittelqualität umfasst, wobei die Zusammensetzung zur internen, externen, parenteralen, topischen, dermatologischen Verwendung oder zur Verwendung auf Geweben, Dermis oder Schleimhaut oder zur oralen, nasalen, okulären Verwendung; oder zur Verwendung im Gehörgang, Rachen, Luftröhre, Speiseröhre, Auge, Nase oder Ohr oder zur Verwendung auf der Mundschleimhaut, der Nasenschleimhaut, dem Nasenepithel, der Augenschleimhaut oder des Gehörgangs oder zur Verwendung im Magen, zur Darm-, Magen-Darm-Verwendung oder zur vaginalen oder rektalen Verwendung dient.

12. Zusammensetzung zur Verwendung nach Anspruch 11, wobei die Zusammensetzung vorliegt:
- in fester Form, ausgewählt aus: Tabletten, Kautabletten, mundlöslichen Tabletten, Granulaten, Pulvern, Flocken, löslichen oder wasserlöslichen Pulvern oder Granulaten, mundlöslichen Pulvern oder Granulaten, Kapseln; oder
- in flüssiger Form, ausgewählt aus: Lösungen, Suspensionen, Dispersionen, Emulsionen, Flüssigkeit, die in Form von Spray, Rachenspray, Mundspray oder Nasenspray, Aerosol, Mundaerosol oder Nasenaerosol, Sirupe abgegeben werden kann; oder
- in halbflüssiger oder halbfester Form, ausgewählt aus: Weichgel, Gel, Nasengel, orales Gel, Salbe, Creme, Creme oder Gelen zur topischen, oralen oder nasalen Verwendung.

13. Zusammensetzung zur Verwendung nach Anspruch 11 oder 12, wobei die Zusammensetzung unter Verwendung mindestens eines akzeptablen Zusatzstoffs und/oder Hilfsstoffs in pharmazeutischer oder Lebensmittelqualität formuliert ist, der ausgewählt ist aus: Kollagen, Chondroitin, Hyaluronsäure oder einem Salz davon, wie Natriumhyaluronat, Vitaminen, Aminosäuren, Antioxidantien, Kieselsäure, Cellulose, Carboxycellulose, Carboxymethylcellulose (CMC), Carboxyethylcellulose, Carboxypropylcellulose, Hydroxypropylcellulose (HPC), Hydroxypropylmethylcellulose (HPMC), Hydroxymethylcellulose, Hydroxyethylcellulose, Hydroxypropylcellulose, Poloxameren, Fasern, Inulin, Maltodextrin, Fetten, Wachsen, Ölen, tierischen Ölen, pflanzlichen Ölen, Vaseline, Carbonat, Bicarbonat, Ammoniumsalzen, Natriumchlorid, Tonen, Kaolin, Antioxidantien, wie beispielsweise Glutathion, Quercetin, Coenzym Q10 oder Vitaminen, insbesondere Vitaminen der Gruppe C oder E, wie beispielsweise Tocopherolen und Tocotrienolen, Tocotrienolen pflanzlichen Ursprungs, wie beispielsweise Tocotrienolen aus Reis, Konservierungsmitteln, wie beispielsweise Natrium- und Kaliumbenzoat, oder Natrium- oder Kaliumsorbat, Mineralsalzen, Natriumchlorid, und pH-Pufferstoffen, wie beispielsweise Zitronensäure und einem löslichen Salz davon, beispielsweise Zitronensäure-Monohydrat.

14. Zusammensetzung zur Verwendung nach einem der Ansprüche 11-13, wobei die Zusammensetzung eine wässrige flüssige Lösung ist, die unter Verwendung eines Zerstäubers oder Sprühers oder einer Sprühdose verabreicht wird; vorzugsweise wird die Zusammensetzung unter Verwendung von Zusatzstoffen und/oder Hilfsstoffen in Lebensmittel- oder pharmazeutischer Qualität formuliert, die es ermöglichen, die flüssige wässrige Zusammensetzung auf Raumtemperatur zu bringen, um eine Konsistenz eines Gels aufzunehmen, sobald die flüssige Zusammensetzung, beispielsweise bei einer Raumtemperatur von etwa 20 °C oder 5 °C, über den nasalen Weg auf ein Subjekt mit einer Körpertemperatur, die beispielsweise von 36 °C bis 40 °C beträgt, aufgetragen wird.

15. Zusammensetzung zur Verwendung nach Anspruch 14, wobei die Zusammensetzung in einer wässrigen Zusammensetzung verwendet wird, die mit Hydroxypropylmethylcellulose (HPMC) und Tocotrienolen, vorzugsweise Tocotrienolen aus Reis, zerstäubt, kombiniert oder assoziiert werden soll; wobei Tocotrienole aus Reis *(Oryza sativa* L.) vorzugsweise in Form eines wasserdispergierbaren Pulvers, beispielsweise mit einem Tocotrienolgehalt von 5 bis 75 Gew.- %, vorzugsweise von 15 bis 60 Gew.- %, beispielsweise 25 Gew.- % oder 30 Gew.- % oder 40 Gew.- % oder 50 Gew.- % vorliegen.

16. Zusammensetzung zur Verwendung nach Anspruch 15, wobei das wasserdispergierbare Pulver zusammen mit den Tocotrienolen, noch bevorzugt Maltodextrin (9050-36-6), Stärke und Siliciumdioxid SiO2 (7631-86-9) enthält; vorzugsweise enthält das wasserdispergierbare Pulver einen Reiskleieölextrakt von 30 % bis 60 %, modifizierte Stärke von 25 % bis 45 %, Maltodextrin von 20 % bis 40 % und SiO2 von 0,2 bis 1,5 %; noch bevorzugter ein wasserdispergierbares Pulver, das eine Mischung aus Tocotrienolen und Tocopherolen in einer Gesamtgewichtsmenge von 15 % bis 60 % enthält, beispielsweise 25 %, oder 30 %, oder 40 %, oder 50 % ist verwendet, noch bevorzugter liegt bei einem Gesamtgehalt in der Mischung von 25 Gew.- % beispielsweise eine Menge von etwa 10 Gew.- % Tocotrienole und eine Menge von etwa 15 Gew.- % Tocopherole vor (Menge gemessen mittels HPLC), während bei einem Gesamtgehalt in der Mischung von 30 Gew.- % etwa 15 Gew.- % Tocotrienole und etwa 15 Gew.- % Tocopherole vorliegen, die restlichen zu 100 % Stärke, Maltodextrinen, SiO2 und anderen vorliegen.

17. Zusammensetzung zur Verwendung nach einem der Ansprüche 11 bis 16, wobei die Usninsäure D(+) einen Schmelzpunkt (DSC) von 190 °C bis 210 °C, vorzugsweise von 192 °C bis 198 °C; eine Molekülrotation von 480° bis 540°, vorzugsweise von 490° bis 520°; einen PSD-Wert: D50 von 1 Mikrometer bis 15 Mikrometer, vorzugsweise von 2 Mikrometer bis 10 Mikrometer, beispielsweise 5 Mikrometer aufweist; noch bevorzugter weist die Usninsäure einen geringen Gehalt an Krankheitserregern wie *Staffilococco Aureus* MIC von weniger als oder gleich 500 ppm und *Escherichia Coli* MIC von weniger als oder gleich 1000 ppm auf.

18. Zusammensetzung zur Verwendung nach einem der Ansprüche 11 bis 17, wobei die Zusammensetzung Folgendes umfasst:
| Inhaltsstoffe | Zusammensetzungen (von x% bis y%) | | | |
|---|---|---|---|---|
| Wasser | 94-99 | 95-98 | 96-97,8 | Wässrige flüssige |
| CMC oder HPC oder HPMC | 0,25-2,5 | 0,5-1,5 | 0,65-0,95 | |
| Usninsäure oder ein Natriumsalz | 0,0001-0,5 | 0,0005-0,05 | 0,001-0,0075 | Lösungen bei 20 °C |
| Beta-Cyclodextrin | 0,005-0,5 | 0,0075-0,25 | 0,01-0,015 | |
| NaCl | 0,1-2 | 0,3-1,5 | 0,5-0,9 | |
| Natriumbenzoat | 0,001-1 | 0,01-0,75 | 0,05-0,1 | |
| Kaliumsorbat | 0,001-1 | 0,01-0,75 | 0,05-0,1 | |
| Tocotrienole oder Mischung aus Tocotrienolen und Tocopherolen | 0,001-1 | 0,01-0,75 | 0,05-0,1 | |
| Zitronensäure | 0,001-0,1 | 0,01-0,005 | 0,015-0,02 | |
| Gesamtgewicht (Gew./Gew.) | 100 | | | |
| Dichte 1 g/cc | | | | |

19. Zusammensetzung zur Verwendung nach einem der Ansprüche 11 bis 18, wobei die Zusammensetzung in Form einer wässrigen Lösung umfasst oder alternativ besteht aus:
- Wasser, vorzugsweise demineralisiertes(m) Wasser, in einer Gewichtsmenge von 90 % bis 99,5 %, vorzugsweise von 94 % bis 99 %;
- eine(r) Einschlussverbindung (ci), wobei die (a) Usninsäure vorzugsweise in der Form D(+), in einer Gewichtsmenge von 0,0001 % bis 0,5 %, vorzugsweise 0,01 bis 0,25 % vorliegt;
- ein(em) Polymer, ausgewählt aus der Gruppe umfassend oder alternativ bestehend aus: Cellulose, Carboxycellulose, Carboxymethylcellulose (CMC), Carboxyethylcellulose, Carboxypropylcellulose, Hydroxypropylcellulose (HPC), Hydroxypropylmethylcellulose (HPMC), Hydroxymethylcellulose, Hydroxyethylcellulose, Hydroxypropylcellulose, Poloxameren, vorzugsweise Hydroxypropylcellulose (HPMC), in einer Gewichtsmenge von 0,25 % bis 2,5 %, vorzugsweise von 0,5 % bis 1,5 %;
- eine(r) Verbindung mit antioxidativer Wirkung, ausgewählt aus der Gruppe umfassend oder alternativ bestehend aus: Glutathion, Quercetin, Coenzym Q10, Vitaminen, insbesondere den Vitaminen der Gruppe C oder E, wie beispielsweise Tocopherolen und Tocotrienolen, vorzugsweise Tocotrienolen pflanzlichen Ursprungs, wie beispielsweise Tocotrienolen aus Reis;
- vorzugsweise in der Industrie üblicherweise gebräuchliche Mineralsalze(n), Konservierungsmittel(n) und pH-Pufferstoff(en) oder Stabilisatoren.

20. Zusammensetzung zur Verwendung nach Anspruch 19, wobei die Zusammensetzung in Form einer wässrigen Lösung einen Viskositätswert bei 20 °C (mPa•Sekunde) von etwa 240 +/- 40; einen pH-Wert von etwa 5 +/- 0,2, einen Dichtewert g/cm3 von etwa 1,01 +/- 0,1 und eine mikrobiologische Belastung (Gesamtbelastung, Hefen, Schimmelpilze, 2 Krankheitserreger (*Staphilococco Aureus* und *Pseudomonas*)) aufweist, vom Typ: Gesamtbelastung <= 100 KBE, Hefen <= 100 KBE, Schimmelpilze <= 10 KBE und Abwesenheit von Krankheitserregern.

## Revendications

1. Mélange M pour une utilisation dans un procédé de traitement d'une infection virale déclenchée ou provoquée par au moins un coronavirus,
dans lequel ledit mélange M comprend ou, en variante, est constitué :
d'un composé d'inclusion (ci), ledit composé d'inclusion (ci) comprenant ou, en variante, étant constitué :
- (i) (a) d'un acide usnique et/ou (b) d'un sel de celui-ci, et
- (ii) d'au moins une cyclodextrine.

2. Mélange M pour une utilisation selon la revendication 1, dans lequel ledit mélange M est destiné à être utilisé dans un procédé de traitement d'infections virales du système respiratoire déclenchées ou causées par un virus SARS-CoV-2 ; de préférence, ledit mélange M est capable d'inhiber la protéine Spike de ladite liaison du virus SARS-CoV-2 avec au moins une protéase SARS-CoV-2 et/ou une protéine Spike de SARS-CoV-2, de préférence avec la protéine Spike RBD du SARS-CoV-2.

3. Mélange M pour une utilisation selon la revendication 1 ou 2,
dans lequel ledit composé d'inclusion (ci) comprend ou, en variante, est constitué :
- (i) dudit (a) acide usnique et/ou un sel de sodium d'acide usnique, et
- (ii) d'au moins une bêta-cyclodextrine.

4. Mélange M pour une utilisation selon l'une quelconque des revendications précédentes, dans lequel, dans ledit composé d'inclusion (ci), ladite (ii) au moins une cyclodextrine, de préférence au moins une bêta-cyclodextrine, et ledit (i) acide usnique et/ou un sel de celui-ci sont dans un rapport pondéral [(ii): (i)] compris de 3:1 à 1:3, de préférence compris de 2:1 à 1:2, plus préférablement compris de 1,5:1 à 1:1,5, et encore plus préférablement étant de 2:1 ou 1:1.

5. Mélange M pour une utilisation selon l'une quelconque des revendications précédentes, dans lequel ledit (a) acide usnique est d'origine naturelle et est une combinaison ou une association (C/A) entre un acide usnique naturel dextrogyre D(+) et un acide usnique naturel lévogyre L(-) ; de préférence, la forme dextrogyre D(+) est comprise de 0,1 à 99,9 % en poids, par rapport au poids total de la combinaison ou de l'association (C/A), et/ou la forme lévogyre L(-) est comprise de 99,9 à 0,1 % en poids, par rapport au poids total de la combinaison ou de l'association, de préférence dans lequel ledit (a) acide usnique d'origine naturelle se présente sous forme racémique à 50 % (+) et 50 % (-), ou est sensiblement égal à 100 % sous forme dextrogyre D(+).

6. Mélange M pour une utilisation selon l'une quelconque des revendications précédentes, dans lequel ledit (a) acide usnique d'origine naturelle est un sel de métal alcalin ou de métal alcalino-terreux ; de préférence, ledit sel d'acide usnique d'origine naturelle est un sel de sodium d'acide usnique D(+).

7. Mélange M pour une utilisation selon l'une quelconque des revendications précédentes, dans lequel ledit (a) acide usnique est constitué essentiellement du composé : acide usnique (+)-, nom chimique 2,6-diacétyl-7,9-dihydroxy-8,9b-diméthyldibenzo[b,d]furan-1,3(2H,9bH)-dione ; acide usnique (+)- issu d'*Usnea* ; n° CAS : 7562-61-0, EC : 231-456-0 ; de préférence, le sel de sodium d'acide usnique est essentiellement constitué du composé : sel monosodique de 2,6-diacétyl-7,9-dihydroxy-8,9b-diméthyldibenzo-1,3(2H,9bH)-dione ; n° CAS : 34769-44-3, EC : 252-204-6 ; de préférence, la pureté dudit (a) acide usnique et/ou dudit (b) un sel d'acide usnique est comprise de 95 à 99,9 %, de préférence de 96 à 99,5 %, et plus préférablement encore de 97 à 98 % ou 99 % (en % poids/poids).

8. Mélange M pour une utilisation selon l'une quelconque des revendications précédentes, dans lequel ledit (a) acide usnique d'origine naturelle et/ou ledit (b) un sel de celui-ci se présentent sous forme solide pulvérulente avec une taille moyenne de particules comprise de 1 à 100 microns, de préférence de 5 à 50 microns, et plus préférablement encore de 10 à 20 microns.

9. Mélange M pour une utilisation selon l'une quelconque des revendications précédentes, dans lequel ledit (a) acide usnique et/ou ledit (b) un sel de celui-ci, et ladite (ii) au moins une cyclodextrine, de préférence au moins une bêta-cyclodextrine, sont dans un rapport pondéral [(a) et/ou (b): (ii)] sensiblement de 1:2 ou de 1:1.

10. Mélange M pour une utilisation selon l'une quelconque des revendications précédentes, dans lequel ledit composé d'inclusion (ci) comprend des particules solides dudit (a) acide usnique, de préférence de l'acide usnique D(+)- sous forme d'énantiomère sensiblement pur, ou d'un sel de celui-ci, dans lequel lesdites particules solides ont une distribution granulométrique moyenne comprise de 0,01 à 50 µm, de préférence comprise de 0,1 à 30 µm, plus préférablement comprise de 0,15 à 20 µm, et encore plus préférablement comprise de 0,2 à 15 µm.

11. Composition pharmaceutique, composition de dispositif médical selon le règlement (UE) 2017/745, composition pour denrées alimentaires destinées à des fins médicales spéciales (DFMS), composition pour nouvel aliment selon le règlement (UE) 2015/2283, ou composition pour des denrées alimentaires destinées à être utilisées dans une méthode de traitement d'une infection virale déclenchée ou causée par au moins un coronavirus, dans laquelle ladite composition pour une utilisation comprend un mélange M selon l'une quelconque des revendications 1 à 10 et au moins un additif et/ou excipient pharmaceutique ou alimentaire acceptable, dans laquelle ladite composition est destinée à un usage interne, externe, parentéral, topique, dermatologique ; ou à une utilisation sur les tissus, le derme ou les muqueuses ; ou à usage oral, nasal, oculaire ; ou à usage dans le conduit auditif, la gorge, la trachée, l'œsophage, l'œil, le nez ou l'oreille ; ou à usage sur la muqueuse buccale, la muqueuse nasale, l'épithélium nasal, la muqueuse oculaire ou celle du conduit auditif ; ou à usage dans l'estomac, l'intestin ou à usage gastro-intestinal ; ou à usage vaginal ou rectal.

12. Composition pour une utilisation selon la revendication 11, dans laquelle ladite composition se présente :
- sous forme solide choisie parmi : des comprimés, des comprimés à mâcher, des comprimés à dissolution buccale, des granulés, de la poudre, des flocons, de la poudre ou des granulés solubles ou hydrosolubles, des poudres ou des granulés à dissolution buccale, des gélules ; ou
- sous forme liquide choisie parmi : les solutions, les suspensions, les dispersions, les émulsions, les liquides pouvant être administrés sous forme de spray, de spray pour la gorge, de spray buccal ou de spray nasal, les aérosols, les aérosols buccaux ou nasaux, les sirops ; ou
- sous forme semi-liquide ou semi-solide, choisie parmi : les gélules molles, les gels, les gels nasaux, les gels buccaux, les pommades, les crèmes, les crèmes ou les gels à usage topique, buccal ou nasal.

13. Composition pour une utilisation selon la revendication 11 ou 12, dans laquelle ladite composition est formulée à l'aide d'au moins un additif et/ou excipient de qualité pharmaceutique ou alimentaire acceptable, choisi parmi : le collagène, la chondroïtine, l'acide hyaluronique ou un de ses sels, tel que le hyaluronate de sodium, les vitamines, les acides aminés, les antioxydants, la silice, la cellulose, la carboxycellulose, la carboxyméthylcellulose (CMC), la carboxyéthylcellulose, carboxypropylcellulose, l'hydroxypropylcellulose (HPC), l'hydroxypropylméthylcellulose (HPMC), l'hydroxyméthylcellulose, l'hydroxyéthylcellulose, l'hydroxypropylcellulose, les poloxamères, les fibres, l'inuline, la maltodextrine, les graisses, les cires, les huiles, les huiles animales, les huiles végétales, la vaseline, le carbonate, le bicarbonate, les sels d'ammonium, le chlorure de sodium, les argiles, le kaolin, les antioxydants tels que par exemple le glutathion, la quercétine, la coenzyme Q10 ou des vitamines, en particulier des vitamines des groupes C ou E, telles que par exemple les tocophérols et les tocotriénols, les tocotriénols d'origine végétale tels que par exemple les tocotriénols issus du riz, des conservateurs tels que par exemple le benzoate de sodium et de potassium, ou le sorbate de sodium ou de potassium, des sels minéraux, le chlorure de sodium, et des tampons de pH tels que par exemple l'acide citrique et un sel soluble de celui-ci, par exemple l'acide citrique monohydraté.

14. Composition pour une utilisation selon l'une quelconque des revendications 11-13, dans laquelle ladite composition est une solution liquide aqueuse qui est administrée à l'aide d'un atomiseur, d'un vaporisateur ou d'une bombe aérosol ; de préférence, ladite composition est formulée à l'aide d'additifs et/ou d'excipients de qualité alimentaire ou pharmaceutique qui permettent, une fois que la composition liquide, par exemple à une température ambiante d'environ 20 °C ou 5 °C, est appliquée par voie nasale à un sujet dont la température corporelle est, par exemple, comprise de 36 à 40 °C, d'amener la composition aqueuse liquide à température ambiante pour qu'elle prenne la consistance d'un gel.

15. Composition pour une utilisation selon la revendication 14, dans laquelle ladite composition est utilisée dans une composition aqueuse destinée à être atomisée, combinée ou associée à de l'hydroxypropylméthylcellulose (HPMC) et à des tocotriénols, de préférence des tocotriénols issus du riz ; de préférence, les tocotriénols issus du riz *(Oryza sativa* L.) se présentent sous la forme d'une poudre hydrodispersable, par exemple avec une teneur en tocotriénols comprise de 5 à 75 %, de préférence de 15 à 60 %, par exemple 25, ou 30, ou 40, ou 50 % en poids.

16. Composition pour une utilisation selon la revendication 15, dans laquelle ladite poudre hydrodispersable contient, outre les tocotriénols, encore de préférence de la maltodextrine (9050-36-6), de l'amidon et du dioxyde de silicium SiO2 (7631-86-9) ; de préférence, la poudre hydrodispersable contient un extrait d'huile de son de riz de 30 à 60 %, de l'amidon modifié de 25 à 45 %, de la maltodextrine de 20 à 40 % et du SiO2 de 0,2 à 1,5 % ; plus préférablement encore, il est utilisé une poudre hydrodispersable contenant un mélange de tocotriénols et de tocophérols dans une quantité totale en poids comprise de 15 à 60 %, par exemple 25, ou 30, ou 40, ou 50 % ; plus préférablement encore, dans le cas d'une teneur totale dans le mélange de 25 % en poids, nous trouvons par exemple une quantité d'environ 10 % en poids de tocotriénols et une quantité d'environ 15 % en poids de tocophérols (quantité mesurée au moyen de la HPLC), tandis que dans le cas d'une teneur totale dans le mélange de 30 % en poids, nous trouvons environ 15 % en poids de tocotriénols et environ 15 % en poids de tocophérols, la partie restante jusqu'à 100 % étant constituée d'amidon, de maltodextrines, de SiO₂ et autres.

17. Composition pour une utilisation selon l'une quelconque des revendications 11 à 16, dans laquelle l'acide usnique D(+) a un point de fusion (ACD) compris de 190 à 210 °C, de préférence de 192 à 198 °C ; une rotation moléculaire comprise de 480 à 540°, de préférence de 490 à 520° ; une valeur PSD : D50 comprise de 1 à 15 microns, de préférence de 2 à 10 microns, par exemple 5 microns ; plus préférablement encore, ledit acide usnique présente une faible teneur en agents pathogènes tels qu'une CIM de *Staphylococcus aureus* inférieure ou égale à 500 ppm et une CIM d*'Escherichia coli* inférieure ou égale à 1 000 ppm.

18. Composition pour une utilisation selon l'une quelconque des revendications 11 à 17, dans laquelle ladite composition comprend :
| Composants | Compositions (de x % à y %) | | | |
|---|---|---|---|---|
| Eau | 94-99 | 95-98 | 96-97,8 | Solutions liquides aqueuses à 20 °C |
| CMC, ou HPC, ou HPMC | 0,25-2,5 | 0,5-1,5 | 0,65-0,95 | |
| Acide usnique ou un sel de sodium | 0,0001-0,5 | 0,0005-0,05 | 0,001-0,0075 | |
| Bêta-cyclodextrine | 0,005-0,5 | 0,0075-0,25 | 0,01-0,015 | |
| NaCl | 0,1-2 | 0,3-1,5 | 0,5-0,9 | |
| Benzoate de sodium | 0,001-1 | 0,01-0,75 | 0,05-0,1 | |
| Sorbate de potassium | 0,001-1 | 0,01-0,75 | 0,05-0,1 | |
| Tocotriénols, ou mélange de tocotriénols et de tocophérols | 0,001-1 | 0,01-0,75 | 0,05-0,1 | |
| Acide citrique | 0,001-0,1 | 0,01-0,005 | 0,015-0,02 | |
| Poids total (p/p) | 100 | | | |
| Densité 1 g/cm³ | | | | |

19. Composition pour une utilisation selon l'une quelconque des revendications 11 à 18, dans laquelle ladite composition, sous forme de solution aqueuse, comprend ou, en variante, est constituée :
- d'eau, de préférence de l'eau déminéralisée, dans une quantité en poids comprise de 90 à 99,5 %, de préférence de 94 à 99 % ;
- d'un composé d'inclusion (ci), dans laquelle ledit (a) acide usnique est de préférence sous la forme D(+), dans une quantité en poids comprise de 0,0001 à 0,5 %, de préférence de 0,01 à 0,25 % ;
- d'un polymère choisi dans le groupe comprenant ou, en variante, constitué de : cellulose, carboxycellulose, carboxyméthylcellulose (CMC), carboxyéthylcellulose, carboxypropylcellulose, hydroxypropylcellulose (HPC), hydroxypropylméthylcellulose (HPMC), hydroxyméthylcellulose, hydroxyéthylcellulose, l'hydroxypropylcellulose, les poloxamères, de préférence l'hydroxypropylcellulose (HPMC), dans une quantité en poids comprise de 0,25 à 2,5 %, de préférence de 0,5 à 1,5 % ;
- d'un composé à activité antioxydante choisi dans le groupe comprenant ou, en variante, constitué de : glutathion, quercétine, coenzyme Q10, vitamines, en particulier les vitamines du groupe C ou E, telles que par exemple les tocophérols et les tocotriénols, de préférence les tocotriénols d'origine végétale tels que par exemple les tocotriénols issus du riz ;
- de préférence de sels minéraux, d'agents conservateurs et de tampons de pH ou de stabilisants couramment utilisés dans l'industrie.

20. Composition pour une utilisation selon la revendication 19, dans laquelle ladite composition, sous forme de solution aqueuse, présente une valeur de viscosité à 20 °C (mPa•seconde) d'environ 240 ± 40 ; une valeur de pH d'environ 5 ± 0,2 ; une valeur de densité g/cm³ d'environ 1,01 ± 0,1 et une charge microbiologique (charge totale, levures, moisissures, 2 agents pathogènes (*Staphylococcus aureus* et *Pseudomonas*)) du type : Charge totale ≤ 100 UFC, levures ≤ 100 UFC, moisissures ≤ 10 UFC et absence d'agents pathogènes.
